# EUROPEAN PATENT APPLICATION

(11) **EP 1 755 098 A2**
(43) Date of publication of application: **21.02.2007**
(21) Application number: 06016569.3
(22) Date of filing: 08.08.2006
(51) Int. Cl.: G09F 19/00, A63B 24/00, A61B 5/00

(54) **Physical rehabilitation systems and methods**

(30) Priority: 08.08.2005 US 199764; 11.10.2005 US 247416
(71) Applicant: BRUNSWICK CORPORATION, Lake Forest, Illinois 60045-4811 (US)
(72) Inventor: Rao, Rajendra, Libertyville Illinois 60048 (US); Indoliya, Deepak, Newark CA 94560 (US); Padanabhan, Ramanath, Singapore 120518 (SG); Ramachandran, Sriram, Singapore 520153 (SG)
(74) Representative: Samson & Partner

(57) **Abstract**

Systems and methods to implement a physical rehabilitation system are disclosed. A disclosed example system includes at a first location an exercise machine and a biometric sensor configured for use by a person. The example system further includes at a second location a computing device communicatively coupled to the exercise machine and the biometric sensor, wherein the computing device is configured to obtain fitness information from the exercise machine and the biometric sensor. The computing device is further configured to provide configuring data to the exercise machine, wherein the configuring data is determined by a medical professional in response to at least one of the obtained fitness information, a medical history of the person, or a medical status of the person..

## Description

### RELATED APPLICATIONS

This application is a continuation-in-part of U.S. Application Serial No. 11/199,764 entitled "Methods and Apparatus for Monitoring Quality of Service for an Exercise Machine Communication Network" and filed on August 8, 2005. U.S. Application Serial No. 11/199,764 is incorporated herein by reference in its entirety.

### FIELD OF THE DISCLOSURE

The present disclosure relates generally to physical rehabilitation systems and, more particularly, to methods and systems to implement physical rehabilitation systems.

### BACKGROUND

The ever increasing concern over personal physical health has motivated many people to partake in various types of health and fitness regimens. Most notably, many individuals join health clubs or physical fitness facilities and/or purchase home exercise equipment with intentions to exercise regularly and, in some instances, follow a specific exercise regimen. People are often drawn to health clubs because of the variety of available exercise machines, exercise equipment, and exercise classes. People may also be drawn to health clubs by the various amenities such as the expertise of health club personnel including personal trainers provided by these facilities. Still further, health clubs may draw people by offering on-site fitness shops where health club members can quickly and conveniently purchase nutritional supplements, dietary consumables, fitness apparel, and other fitness-related products.

Some people prefer to purchase home exercise equipment and exercise in the privacy of their home. For example, if a person wants to build cardiovascular endurance and trim body fat, that person may purchase a treadmill, an elliptical trainer, a stepper, and/or any other suitable cardiovascular machine and have the cardiovascular machine installed in their home. A person that wants to strength train may purchase a home weight training system such as a universal gym. In some cases, people that prefer to exercise at home hire personal fitness trainers who make house calls on a periodic basis to offer exercise-related and nutritional-related advice or guidance to clients and to assist their clients in performing exercises.

Other people may decide to join a fitness club and purchase home exercise equipment. For example, a person may join a fitness club at or near their work place to have access to a convenient place to exercise during the week and to provide the person with all the amenities available at the fitness club environment that are not available in a home gym environment. The person may then use the home exercise equipment during the weekend or at times when it is more convenient to exercise at home rather than at the fitness club. In some cases, a person that travels frequently may elect to purchase home fitness equipment to use when the person is not traveling, but also joins a fitness club chain having facilities in many of the destinations to which the person usually travels. In this manner, the person may conveniently exercise whether at home or traveling.

Despite people's intentions to follow exercise regimens when they initially decide to join a fitness club and/or decide to purchase home exercise equipment, studies show that people tend to stray from their goals and many cease to exercise as once intended. For example, one study indicates that the average duration of a fitness club membership is sixty-eight weeks, and that fitness clubs experience attrition rates that range from about 30% to about 50%. Studies such as these indicate losses for the fitness clubs and for members who cease to exercise and/or cancel their fitness club membership. To mitigate the negative affects that membership cancellations have on the financial condition of fitness clubs, fitness clubs may increase membership fees and fitness-related product fees, and cancel certain amenities or services. However, in some cases, fitness clubs unable to mitigate the financial impact of membership cancellations are forced to close.

Fitness club members who cease to exercise or decide to cancel their memberships often stray toward sedentary and/or unhealthy lifestyles. Although people often make numerous excuses for why they stray from their regular exercise routines, often the reason for people's waning of regular exercise is a lack of guidance or motivation. For example, a person that vows to follow a strict exercise regimen may stray because of a lack of prodding or coaching. Some people need at least some level of outside guidance or encouragement to stay on track with their exercise program(s). However, a person may not be able (e.g., due to financial constraints) to hire a personal trainer and, in some cases, fitness clubs don't have sufficient personal trainers to meet the demands of their members. Thus, members eventually become discouraged and quit when they don't receive the attention, encouragement, or guidance required to stay motivated.

Another issue often related to the ability of a fitness club to retain members is related to the quality of the fitness equipment. To remain competitive, fitness clubs equip their facilities with the latest electro-mechanical exercise machines. The software, hardware, and mechanical complexities of these exercise machines provide many opportunities for equipment failure. For example, as is well known, any electronic and/or software driven device is subject to electronic malfunctions and/or software bugs that may render the device inoperable. Also, the continuous and repetitive use of exercise machines imparts significant wear to the mechanical components of the exercise machines and eventually causes the exercise machines to fail and become inoperative until serviced. Club members typically have little patience for inoperable equipment, especially when exercise machines are continuously inoperable or remain unserviced for days. Further, member frustrations related to inoperable exercise equipment often rise during peak hours (e.g., early morning or after work) when availability of machines is scarce due to the large number of members within the fitness club. Members that become dissatisfied with equipment maintenance often cancel their memberships and take their business elsewhere, exercise at home, or quit their exercise regimens entirely.

### BRIEF DESCRIPTION OF THE DRAWINGS

FIG. 1 is an example fitness network system that communicatively couples a plurality of locations to exchange fitness information therebetween.

FIG. 2 depicts a plurality of example entities that may be communicatively coupled to the example fitness network system of FIG. 1 to exchange fitness information with a data center.

FIG. 3 is a detailed block diagram of an example exercise machine control system that may be implemented in connection with an example exercise machine to implement at least some of the example methods and systems described herein.

FIG. 4 is a flowchart of an example method that may be used to store fitness information in a data record associated with a fitness member account.

FIGS. 5A and 5B depict a flowchart of an example method that may be used to implement operations of an exercise machine and a server that are communicatively coupled to the example fitness network system of FIG. 1.

FIG. 6 is a flowchart of an example method that may be used to implement a fitness-related messaging application on a portable computing device that is communicatively coupled to the fitness network system of FIG. 1.

FIG. 7 is a flowchart of an example method that may be used to monitor the operating status of an exercise machine that is communicatively coupled to the example fitness network system of FIG. 1.

FIG. 8 is a block diagram of an example processor system that may be used to implement the example methods and systems described herein.

FIG. 9 is an example template generator user interface that may be used to generate exercise program templates.

FIGS. 10 and 11 are example template user interfaces depicting exercise program templates associated with exercise machines.

FIGS. 12 and 13 are example exercise machine console user interfaces associated with exercise machines communicatively coupled to the example fitness network system of FIG. 1.

FIGS. 14 and 15 are example exercise machine monitoring user interfaces associated with exercise machine monitoring processes.

### DETAILED DESCRIPTION

Although the following discloses example systems including, among other components, software and/or firmware executed on hardware, it should be noted that such systems are merely illustrative and should not be considered as limiting. For example, it is contemplated that any or all of these hardware, software, and firmware components could be embodied exclusively in hardware, exclusively in software, or in any combination of hardware and software. Accordingly, while the following describes example systems, persons of ordinary skill in the art will readily appreciate that the examples provided are not the only way to implement such systems.

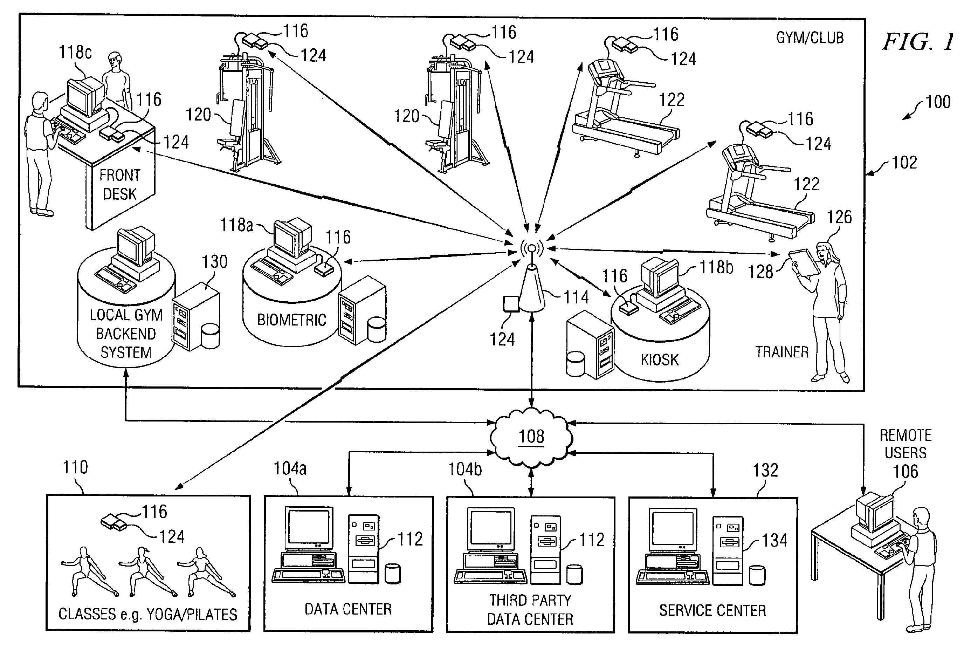

The example systems and methods described herein can be used to implement an example fitness network system (e.g., the example fitness network system 100 of FIG. 1) that includes a plurality of communicatively coupled network servers and network access nodes (e.g., network communication devices, data terminals, etc.) to store fitness-related information associated with members and fitness facilities, and can be used to exchange or convey the fitness-related information between the servers and nodes. Fitness-related information (i.e., fitness information) may include member profile information, attendance history information, strength information, cardiovascular information, nutritional intake, health condition information (e.g., blood pressure, injuries, etc.), physiological conditions (e.g., heart rate), workout schedules, and/or any other health or fitness-related conditions associated with a person. The example fitness network system 100 enables fitness members (e.g., fitness club members, physician patients, rehabilitation program members, sports team members, etc.) to follow a fitness program (e.g., a training program, a conditioning program, a rehabilitation program, etc.), log and monitor fitness information, store the fitness information, and access the fitness information from a plurality of access nodes and locations. In this manner, fitness members and interested, authorized third parties (e.g., fitness trainers, physicians, insurance companies, etc.) can monitor or obtain health status or fitness status information of the fitness members.

The example fitness network system 100 is communicatively coupled to the Internet or to any wide area network (WAN) to enable access to the fitness information from a plurality of locations some or all of which may be relatively geographically distributed. The fitness network system 100 enhances an overall fitness program experience by providing access to fitness information within a fitness club via, for example, kiosks and exercise machines, and outside the fitness club via, for example, other fitness clubs, home computers, home gym equipment, physician offices, physical therapy facilities, insurance companies, etc. Persons may access the fitness information using web-based applications installed on data terminals communicatively coupled to the fitness network system 100. The fitness network system 100 also includes a plurality of tools and templates to enable a person to select the type of fitness information to access and to customize or personalize the manner in which the fitness information is accessed or viewed. For example, a fitness member may want to access all of the fitness information that is logged about the fitness member such as, for example, exercise progress, fitness goals, personal trainer recommendations, workout schedules, health status information, etc., while a physician may only want to access health status information and exercise progress and an insurance company may only want to access health status information. Each user can customize their information access to retrieve and/or modify only the information that is of interest to that user. In some examples, access to certain types of fitness information may be limited so that users may be authorized to retrieve only certain fitness information regardless of their interests.

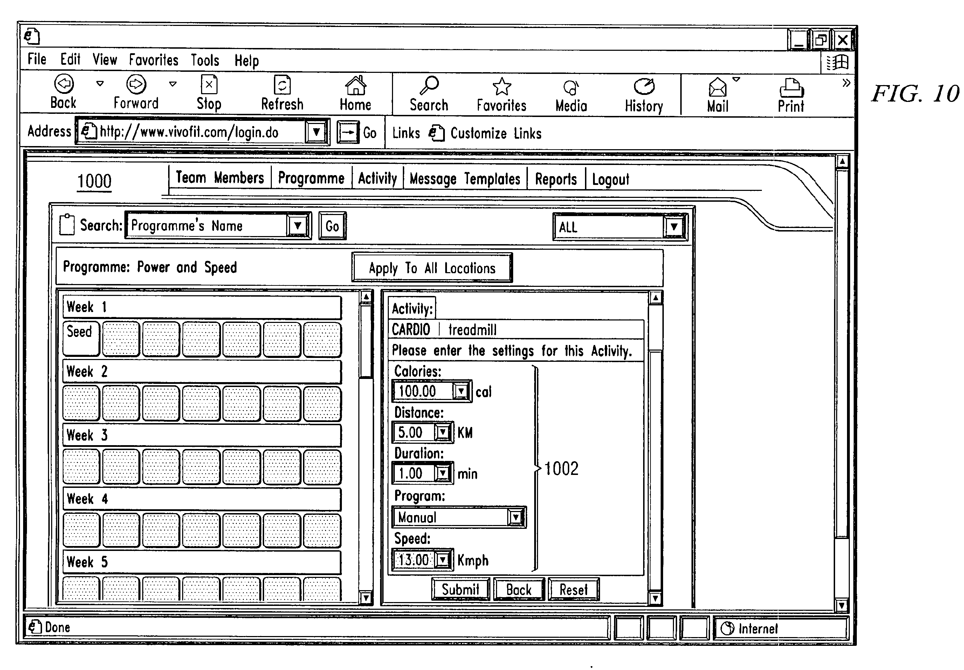

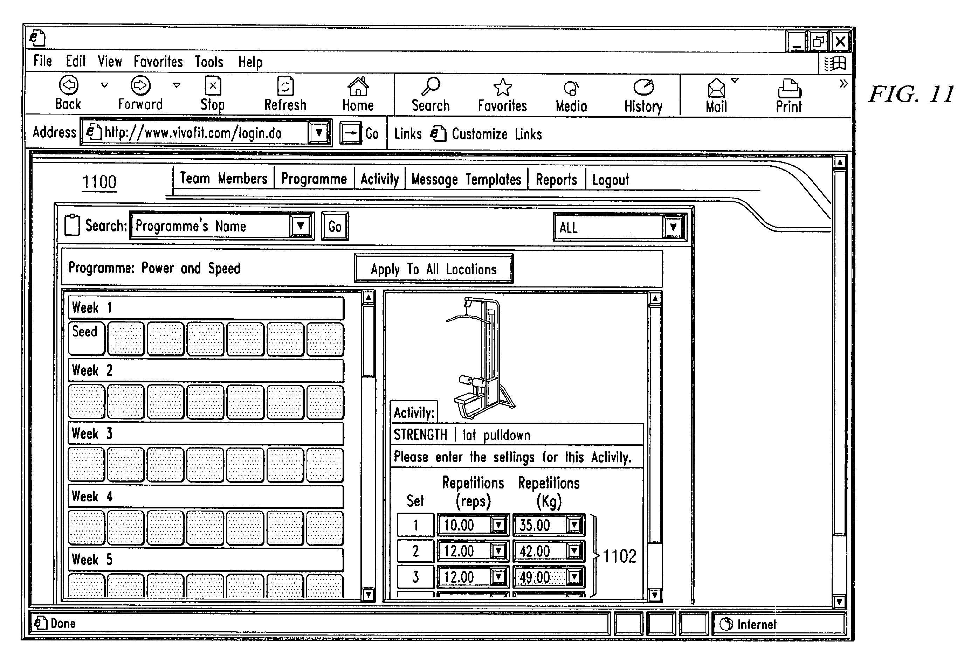

Terminals (e.g., computers) communicatively coupled to the fitness network system 100 also include a plurality of templates (e.g., example template user interfaces 1000 and 1100 of FIGS. 10 and 11) that enable users to create workout programs. The templates may include templates directed toward particular fitness goals (e.g., cardiovascular training, strength training, physical therapy or rehabilitation training, marathon training, weight loss, sports training, etc.). The templates may include a plurality of parameters that guide a user (e.g., a fitness member, a personal trainer, a physical therapist, etc.) to create workout routines based on individual fitness member capabilities. For example, a physical therapy template may include minimum and maximum-type parameters that restrict the amount of physical exertion during particular exercises or that restrict settings for particular exercise machines. The physical therapy template may restrict the amount of weight resistance that can be set for a leg extension machine if a fitness member is participating in physical therapy sessions for a knee injury. An example marathon training template may restrict a running duration or the speed at which a fitness member should run on a treadmill during particular days of a marathon training program.

Exercise machines (i.e., exercise devices) within a fitness club, a home, a rehabilitation center, or any other location may be communicatively coupled to the example fitness network system 100. For example, exercise machines within a fitness club may be wirelessly communicatively coupled to wireless access points that provide access to other processor systems and data storage devices of the fitness network system 100. An exercise machine may include a plurality of machine sensors and physiological sensors that acquire machine measurement data (e.g., speed) and physiological measurement data that is then communicated to a server. Machine measurement data may be associated with a person's exercise routine (e.g., speed, incline, repetitions, resistance, etc.) or may be associated with operating performance of electrical, mechanical, or software subsystems of the exercise machine. The physiological sensors may be used to measure a person's physiological conditions (e.g., heart rate) during exercise.

The exercise machines may be configured to identify a member based on a radio frequency identification (RFID) device sensor, user input (e.g., member identification number or code) a card having a magnetic strip or barcode, etc. In this manner, the exercise machines can retrieve exercise machine programs, workout schedules, and workout goals for each fitness member via the example fitness network system 100 based on a member identification code. Also, the fitness network system 100 can store updated or new fitness information for each fitness member based on the member identification code.

Each exercise machine that is communicatively coupled to the fitness network system 100 may also include diagnostics hardware or software to track or log service information such as, for example, machine functionality and machine usage information. The machine functionality information may include software operability, hardware operability, mechanical operability, etc. For example, the exercise machines may include diagnostics software that periodically or in response to predefined events (e.g., power on or power off) performs a diagnostics error checking routine of software and electrical systems to ensure that there are no malfunctions or other errors in the electrical and software systems. If the diagnostics software detects an error, the exercise machine may generate a maintenance ticket or a trouble ticket and communicate the maintenance ticket to a service center server via the fitness network system 100. Machine usage information may include maximum, minimum, and average speed settings that users typically select, the frequency of machine use in a typical day, peak usage times, etc. The machine usage information may periodically be communicated to a service center server and used to determine improvements for future developments of, for example, fitness club services and exercise machines.

The example fitness network system 100 may also be used to log fitness information associated with exercise or workout routines performed without an exercise machine. For instance, a person may use a kiosk at a fitness facility, a home computer, or a network-enabled portable computing device (e.g., a personal digital assistant (PDA), a mobile phone, a computing tablet, etc.) to enter fitness information related to exercise classes (e.g., yoga, aerobics, etc.), outdoor activities (e.g., hiking, running, cycling, swimming, etc.), sports participation (e.g., racquetball, tennis, soccer, etc.), or any other fitness activities for which electronic logging capabilities are unavailable.

The example fitness network system 100 also includes a messaging system to provide messages to fitness members and enable fitness members to interact with personal trainers and fitness facility personnel. In this manner, personal trainers may assist fitness members and answer questions even when the personal trainers' or the fitness members' schedules do not allow meetings, sessions, or face-to-face conversations. Messages may include motivational or guidance information sent to the fitness members by personal trainers or other fitness facility personnel to keep fitness members motivated and on track and to help the fitness members attain their fitness goals. The messages may also include general fitness related messages such as, for example, exercise tips, nutritional tips, health news, etc. The messaging system may also enable the fitness facility to send membership account related information to the fitness members. Also, the fitness facility may send advertisement messages to fitness members regarding, for example, fitness facility services, products at the fitness facility shop, membership upgrades, etc.

In an example implementation, the fitness network system 100 may log fitness members' activities and/or habits and the messaging system may generate messages based on the logged activity information and date information obtained from an electronic clock and/or calendar. For example, if log activity information indicates that a fitness member often uses elliptical exercise machines, the messaging system may provide a message to the fitness member indicating when new elliptical machines have arrived or are scheduled to arrive to a fitness facility. Additionally, if a fitness member's logged activities indicate that the member is associated with a particular group (e.g., a sports team, a running club, etc.), the messaging system may send messages to inform the fitness member when events (e.g., games, meets, training sessions, group events, social gatherings, etc.) associated with that group are scheduled.

The example fitness network system 100 also enables interactive messaging between fitness members and personal fitness trainers and/or any other third party (e.g., physicians, physical therapists, health professionals, insurance companies, fitness instructors, sports coaches, etc.). For example, the fitness network system 100 may send progress updates (e.g., strength training progress, cardiovascular progress, health status, nutritional intake, etc.) of a fitness member to the fitness member's personal trainer. In this case, the personal trainer may carry or wear a wireless-enabled communication device (e.g., a PDA, a mobile phone, a PC tablet, etc.) capable of receiving communications from the fitness network system 100. The personal trainer may assess the progress updates and send messages (e.g., e-mail messages) to the fitness member regarding new goals, exercise routines, suggested nutritional intake, motivational messages, etc. based on the progress updates. A fitness member may also send questions to a personal trainer via any data terminal communicatively coupled to the fitness network system 100.

The fitness network system 100 may also be used to track or monitor fitness member attendance history. In one example implementation, a fitness club may include member identification sensors (e.g., RF sensors) communicatively coupled to the fitness network system 100 to determine when fitness members are present within the fitness club. The fitness network system 100 may alert personal trainers via wireless-enabled communication devices when any of their fitness members enters the fitness club. In some examples, the fitness network system 100 may alert personal trainers when their fitness members are using particular exercise machines. Of course, the fitness network system 100 may also communicate alerts to personal trainers via wireless-enabled communication devices in response to a fitness member entering information (e.g., a membership ID) or swiping a membership card at a kiosk or other computer terminal within the fitness club. Alerts may also be communicated to personal trainers based upon one or more rules associated with various conditions. For example, an alert may be communicated to the personal trainer after a particular number of visits to a fitness facility by a fitness member, when the fitness member exceeds a pre-determined workout limit on a cardio-vascular and/or a strength machine (e.g., a time limit, a distance limit, a resistance limit, a calories burned limit, etc.), when the fitness member does not follow a pre-determined workout sequence, etc. In the fitness network system 100, the rules may be customizable for each fitness member.

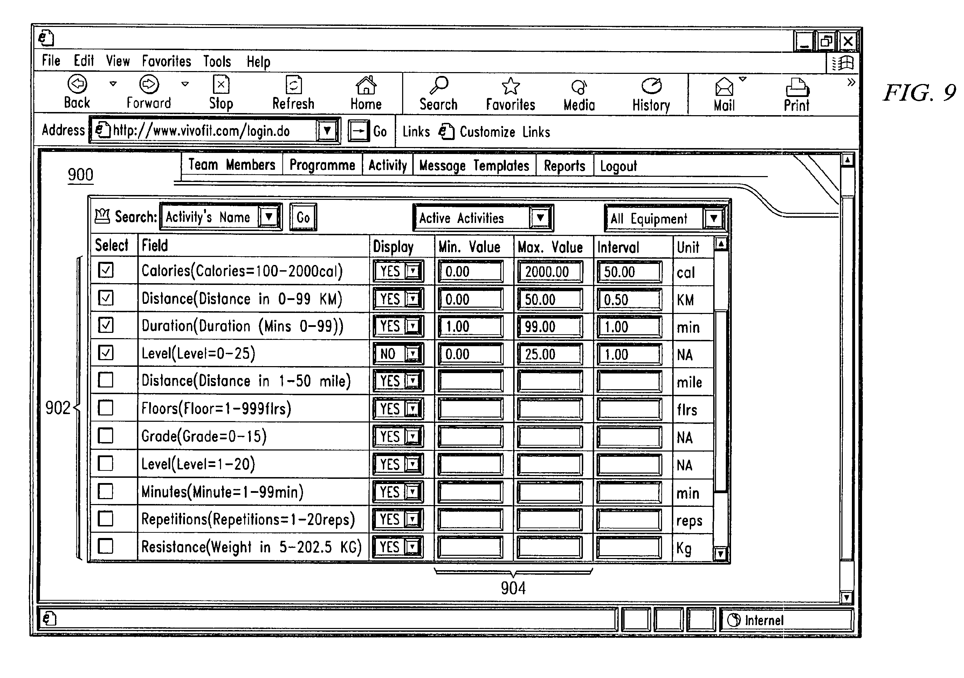

Now turning in detail to FIG. 1, the example fitness network system 100 communicatively couples a plurality of locations to exchange fitness information therebetween. As shown in the illustrated example, the fitness network system 100 communicatively couples a fitness facility 102, data centers 104a and 104b, and a remote computer 106 via a communication network 108. The communication network 108 may be implemented using the Internet or any WAN such as, for example, one or more digital subscriber lines (DSL's), an integrated services digital network (ISDN), and/or any other broadband networking technology. In the illustrated example, the fitness facility 102 is a gym or fitness club having exercise equipment and a studio classroom 110 for fitness classes (e.g., yoga, aerobics, etc.). The data centers 104a-b include one or more servers 112 configured to include databases or data structures that store fitness information. In this example, the servers 112 are configured to provide access to the fitness information via the communication network 108 and may be implemented based on the example processor system 810 of FIG. 8. Although the servers 112 are shown at locations separate from the fitness facility 102, in an alternative implementation, the servers 112 may be located within the fitness facility 102.

The remote computer 106 may be any computer terminal outside of (e.g., remotely situated relative to) the fitness facility 102 such as, for example, a home computer, a school computer, a work computer, a mobile phone, a PDA, etc. In general, the remote computer 106 may be implemented using any processor system (e.g., the example processor system 810 of FIG. 8) that can communicatively access the communication network 108. The remote computer 106 may exchange fitness information with the servers 112 using, for example, web-based applications, file transfer protocol (FTP) applications, etc. using synchronization logic. In one example implementation, the remote computer 106 may include a locally installed client application that stores fitness information in a local memory (e.g., a mass storage memory 825 of FIG. 8) within the remote computer 106 and that periodically or aperiodically synchronizes the fitness information stored in the local memory with fitness information stored in the servers 112. In an alternative implementation, the fitness information in the servers 112 may be accessed via the remote computer 106 using, for example, a web browser application.

A fitness member may use the remote computer 106 to specify health or exercise goals or goals associated with or specific to particular exercise activities or programs. The fitness member may also create or modify workout schedules, view fitness progress, enter nutritional intake information, exchange messages with fitness facility staff or personal trainers, and print some or all of the available fitness information. Of course, any other type of user including, for example, fitness facility staff, personal trainers, physicians, physical therapists, etc. may access fitness information stored at the servers 112.

The remote computer 106 enables a fitness member or any other user to prepare a workout program or schedule prior to the fitness member exercising so that the fitness member can access fitness information associated with that workout program while the fitness member is within the fitness facility 102. Specifically, any fitness information changed or added by a user via the remote computer 106 may be retrieved from the servers 112 by a user from any processor system or exercise machine within the fitness facility 102. For instance, fitness information provided to the servers 112 by a fitness member via the remote computer 106 may be retrieved by an exercise machine console associated with any of the exercise machines 120 and 122 within the fitness facility 102 to enable the exercise machines 120 and 122 to operate according to the fitness member's workout program or schedule.

Although one remote computer is shown, any number of remote computers may be communicatively coupled to and configured to access the fitness network system 100. In one example implementation, an instructor, a manager, a personal trainer, an administrator, a physical therapist, a physician, etc. may create class schedules from a remote computer substantially similar or identical to the remote computer 106. Fitness members may then access the class schedules via other remote computers and/or computers within the fitness facility 102 to enroll in classes based on the class schedules.

Although one fitness facility (i.e., the facility 102) is shown in the illustrated example of FIG. 1, the fitness network system 100 may communicatively couple any number of fitness facilities to one another and to the data centers 104a-b. In this manner, fitness information may be exchanged between any fitness facility and the data centers 104a-b. In particular, a fitness chain may enable its members to access their fitness information from any of the fitness facility locations or any affiliated fitness facilities (e.g., hotel fitness facilities, physical therapy facilities, etc.) that are associated with the fitness chain and communicatively coupled to the fitness network system 100. In this manner, a member can access fitness information stored in the servers 112 while visiting any fitness facility. That is, an exercise machine console at any fitness facility communicatively coupled to the fitness network system 100 can retrieve fitness information from the servers 112 when the exercise machine detects a fitness member having a membership ID. Alternatively, the servers 112 may push fitness information to an exercise machine console that reports a membership ID to the servers 112.

The fitness network system 100 includes one or more wireless network access points substantially similar or identical to a wireless network access point 114 within the fitness facility 102. The wireless network access point 114 is configured to communicatively couple a plurality of network devices 116 within the fitness facility 102 to the communication network 108. The wireless access point 114 and the network devices 116 may be implemented using any wireless network access technology such as, for example, IEEE 802.11, Bluetooth®, Zigbee®, or any other suitable wireless networking protocol. Some of the network devices 116 communicatively couple a plurality of computer terminals 118a-c to the wireless network access point 114. In the illustrated example, the plurality of computer terminals 118a-c includes a biometric terminal 118a, a kiosk terminal 118b, and a fitness administrator terminal 118c. The biometric terminal 118a may include a plurality of biometric or physiological sensors (not shown) used to acquire physiological information. For example, a member may periodically (e.g., daily, weekly, etc.) measure resting heart rate, blood pressure, etc. at the biometric terminal 118a, and the biometric terminal 118a may communicate the physiological information to, for example, one of the data centers 104a-b for storage. Fitness members may use the kiosk 118b to receive and send messages to, for example, their personal trainers, physicians, etc., and to view or otherwise access or interact with their fitness information. For example, a fitness member may modify a workout schedule, enter information associated with fitness activities (e.g., sports, studio classes, physical rehabilitation sessions, etc.), enter nutritional intake, etc. Fitness members may use the kiosk terminal 118b to access fitness information in a manner that is substantially similar or identical to that described above in connection with the remote terminal 106.

Authorized fitness facility employees may use the fitness administrator terminal 118c to access fitness information and fitness facility-related information. Fitness facility-related information includes, for example, exercise machine inventory, exercise machine maintenance information, class scheduling information, non-machine-based activity information (e.g., outing events, sports events, etc.), membership information, member profile information, broadcast messages, employee messages, etc. Employees can also use the fitness administrator terminal 118c to run reports such as, for example, member activity, equipment usage, facility usage, class attendance, peak utilization times, workout program popularity, frequency of member visits, etc. Fitness facility staff can also use the fitness administrator terminal 118c to perform administrative tasks such as, for example, schedule rotation of equipment, schedule equipment maintenance, change fitness schedule timetables or information, and assign tasks to staff members. In some example implementations, administrative tasks may be performed only from a central terminal or from only one terminal (e.g., the fitness administrator terminal 118c) and may be performed only by users having sufficient permissions or privilege levels based on, for example, passwords or staff ID.

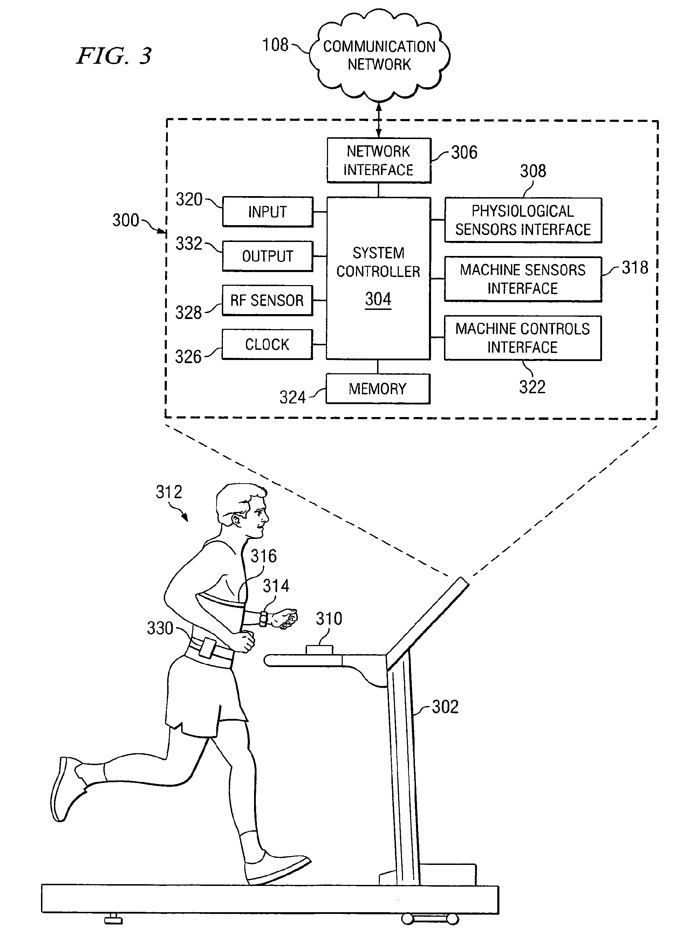

Some of the network devices 116 communicatively couple exercise machines 120 and 122 to the wireless network access point 114. Each of the exercise machines 120 may include a control system (e.g., a hardware system or a processor system) configured to monitor physiological information associated with users of the exercise machine and usage of the exercise machine. For example, a control system (e.g., the control system 300 of FIG. 3) may be configured to acquire physiological information via physiological sensors and communicate the physiological information to the data centers 104a-b via the network 108. The control system may also monitor repetitions, resistance, speed, incline, and other machine settings or machine usage information associated with a fitness member's workout. Also, the control system may monitor machine functionality to ensure that the machine is functioning properly and may acquire and/or generate statistical information associated with machine usage. Of course, the control system could communicate the acquired raw data associated with the machine functionality to a server (e.g., the server 112, a fitness club server, a service center server, etc.) and the server may generate analyses or statistical information. Additionally or alternatively, machine sensors may communicate machine usage-related data directly to a server.

RFID device sensors 124 may be located throughout the fitness facility 102 and communicatively coupled to the network devices 116 or the network access point 114. The RFID device sensors 124 detect RFID device's worn or carried by fitness members to determine when fitness members are within the fitness facility 102. RFID device sensors 124 associated with the exercise machines 120 and 122 may be used to detect and identify fitness members using particular exercise machines. An RFID device sensor 124 located in the studio classroom 110 is used to detect and identify fitness members that participate in workout classes. The RFID device sensor 124 can communicate member ID information to the data centers 104a-b to update members' fitness information to indicate the classes in which members participate to, for example, update or generate attendance history information. Additionally or alternatively, a card reader device (e.g., a magnetic strip reader, a barcode reader, etc.), a user input device (e.g., a computing terminal), a biometric sensor (e.g., a thumbprint reader), etc. may be located in the studio classroom 110 and used to communicate member ID information to the data centers 104a-b.

A personal trainer 126 may access the fitness network system 100 using a wireless-enabled portable computing device 128 (i.e., a portable data device), which may be implemented using, for example, a PDA, a mobile phone, a PC tablet, etc. The personal trainer 126 may use the portable computing device 128 to access, view, and/or modify fitness information. For example, the personal trainer 126 may use the portable computing device 128 to view, modify, or create workout schedules for members, create custom exercise machine programs with or without using pre-defined program templates, exchange messages (e.g., e-mail) with fitness members, etc. Of course, the personal trainer 126 may access some or all of the fitness information using any other computer terminal (e.g., the kiosk terminal 118b, the fitness administrator terminal 118c, the remote terminal 106, etc.) communicatively coupled to the fitness network system 100. The personal trainer 126 may also use the portable computing device 128 to receive notifications based on information from the RFID device sensors 124 when a fitness member is in the fitness facility 102 and/or when the fitness member is using a particular exercise machine.

RFID devices may also be worn or carried by a professional trainer. Thus, any of the wireless-enabled portable computing device 128, the kiosk terminal 118b, the administrator terminal 118c, and/or the remote terminal 106 that have an RFID device sensor 124 can identify any person within detection proximity that is carrying or wearing an RFID device.

In an example implementation, the portable computing device 128 may include a guided interview application installed thereon. The personal trainer 126 may use the guided interview program to obtain information about fitness members and to create workout schedules and/or enroll fitness members in classes via the portable computing device 128. Also, the guided interview application facilitates educating personal trainers about how to interview fitness members and how to create effective workout programs that are customized to meet the needs of each fitness member. The guided interview application may include a list or a plurality of interview questions that a personal trainer 126 may use to assess a fitness member's health, fitness condition level, exercise capabilities, fitness goals, etc. Interview questions may be periodically updated by periodically synchronizing the portable computing device 128 with the fitness network system 100. In this manner, the interview questions may remain current based on any new developments in the health industry. An example guided interview application may include an automatic workout program generation feature that generates workout schedules, nutritional guidance, fitness goals, etc. based on fitness member information.

In the fitness facility 102, the fitness network system 100 includes a local facility backend server 130 that is communicatively coupled to the communication network 108. The local facility backend server 130 may be implemented based on the example processor system 810 of FIG. 8, and may be used to locally store general information about the fitness facility 102. For example, the backend server 130 may be configured to store and monitor class schedules associated with fitness classes held in the studio classroom 110 and to broadcast messages to fitness members when fitness classes are close to starting. The backend server 130 may broadcast class starting messages to the exercise machines 120 and 122 that are being used by fitness members that are interested in those classes. More specifically, in an example embodiment, the backend server 130 may determine what members are using which of the exercise machines 120 and 122 based on member ID information obtained from the RFID device sensors 124, cross-references the member ID information with member profile information or member workout schedules retrieved from local storage or from the data centers 104a-b, and broadcast class starting messages only to the ones of the exercise machines 120 and 122 that are being used by members having an interest in the classes. The backend server 130 may also be used to store exercise machine programs for the exercise machines 120 and 122 and configured to communicate those exercise machine programs to the exercise machines 120 and 122. Of course, the exercise machine programs may additionally or alternatively be stored at the data centers 104a-b.

The backend server 130 may also be used to store advertising messages and other informative messages (e.g., diet tips, exercise tips, notices of new equipment, hours of operation, special events, etc.) that are occasionally broadcast to members. The backend server 130 may be configured to broadcast the advertising or other informative messages to members based on member profiles. For example, the backend server 130 may retrieve member profiles from local storage or from the data centers 104a-b and use the member profile information to determine the types of interests of each fitness member and broadcast messages to fitness members based on their interests. For example, the backend server 130 may broadcast muscle building dietary supplement advertisements to only those members that indicate in their profiles an interest in building muscle mass. The messages may, for example, be sent in e-mail format. In some example embodiments, advertising and other informative messages may additionally or alternatively be sent by the servers 112 in the data centers 104a-b in substantially the same manner. The backend server 130 may also be configured to monitor operations of the various computing systems within the fitness facility 102 and to perform typical backend server operations that are well known in the art.

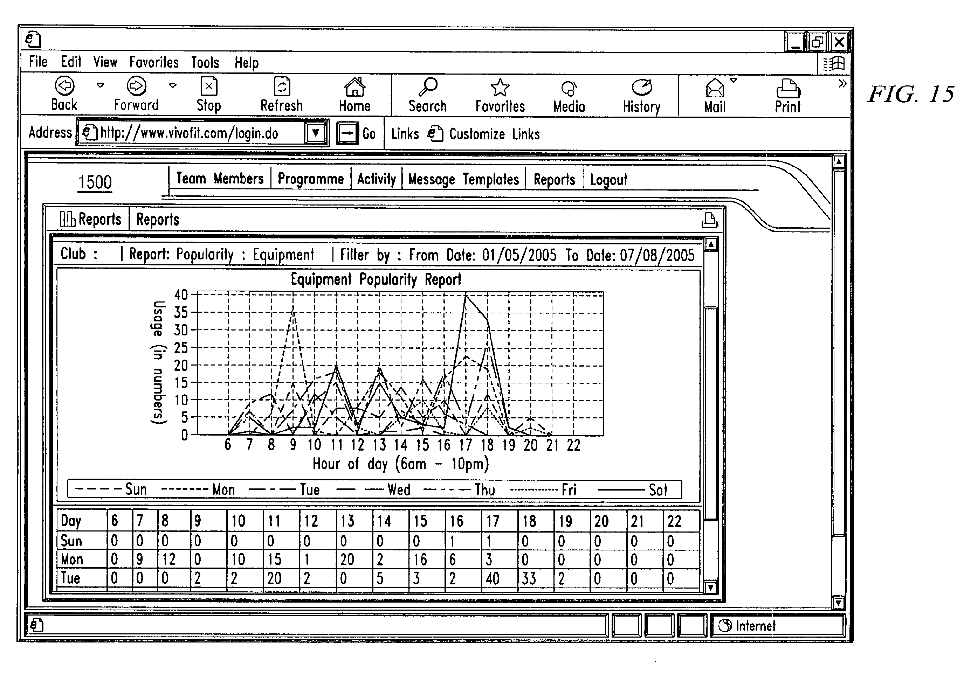

Also shown in FIG. 1 is a service center 132 having a service center server 134 communicatively coupled to the communication network 108. The service center server 134 is configured to store and analyze operating performance information and usage information associated with the exercise machines 120 and 122. The service center server 134 may also communicate queries to the exercise machines 120 and 122 requesting updates associated with the operations and conditions of the exercise machines 120 and 122. In the illustrated example, each of the exercise machines 120 and 122 includes diagnostics hardware and/or software that track or log service information such as, for example, machine functionality and machine usage information (e.g., maximum, minimum, and average speed settings that users typically use, the frequency of use in a typical day, peak usage times, etc.). The exercise machines 120 and 122 are configured to communicate the service information to the service center 132 periodically and/or upon request. The exercise machines 120 and 122 may also be configured to communicate maintenance information such as, for example, maintenance requests or maintenance tickets to the service center 132 in response to predefined events associated with servicing the exercise machines 120 and 122 (e.g., an error event, a malfunction event, etc.). The exercise machines 120 and 122 are also configured to detect improper usage of the exercise machines 120 and 122 and communicate a message associated with the improper usage to the service center server 134 and/or the fitness administrator terminal 118c. In an example implementation, the service center server 134 may monitor frequency of usage for each of the exercise machines 120 and 122 within the fitness facility 102 and display analyses information (e.g., via example exercise machine monitoring user interface 1500 of FIG. 15) to a service technician.

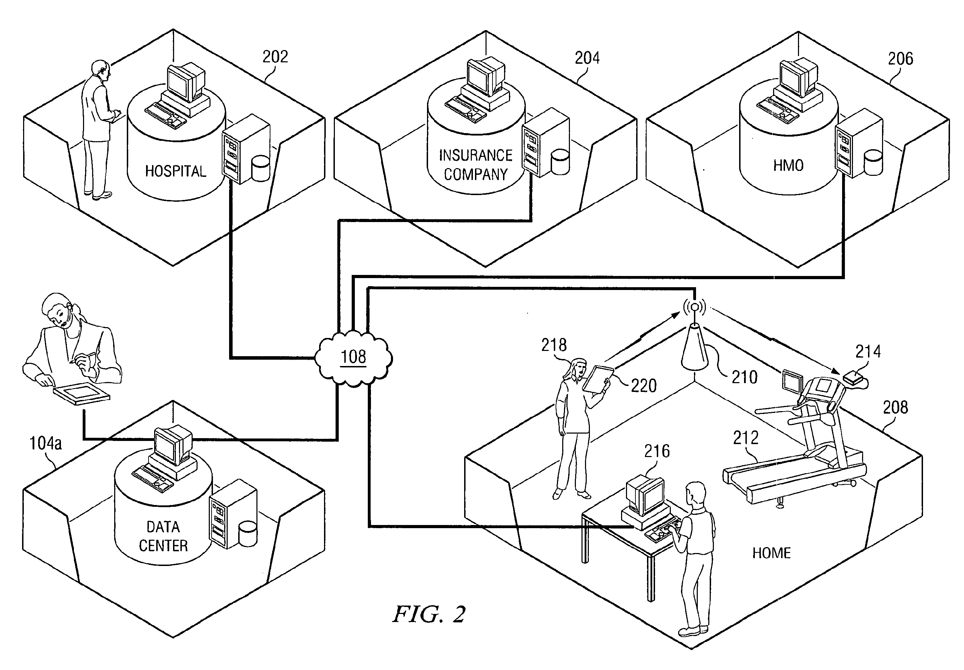

Although the exercise machines 120 and 122 are shown to be located within the fitness facility 102, exercise machines that are communicatively coupled to the service center 132 may be located at any other location. For example, exercise machines configured to exchange service information with the service center 132 may be located in a hotel fitness club, a physical therapy facility, a hospital, a home (e.g., the home 208 of FIG. 2). In some example implementations, service information may include notifications to members about new equipment (e.g., exercise machines) that have recently become available at particular facilities (e.g., the fitness facility 102) and/or may include notifications to members about machines that are out of service or have been removed from operation.

The service center server 134 is configured to monitor and maintain a history of the service information including machine usage information and maintenance requests associated with each of the exercise machines 120 and 122. The server 134 may process or analyze the machine information to determine improvements for future developments of, for example, fitness club services and exercise machines. The server 134 may also be configured to analyze the machine usage information to determine if there has been any improper usage (e.g., abuse) of the exercise machine and communicate a message to fitness facility personnel associated with the improper usage. In some example implementations, the service center 134 (and/or the backend server 130) may also be configured to monitor network quality of service (QoS) associated with the fitness network system 100 as described in detail in U.S. Patent Application Serial No. 11/199,764, filed on August 8, 2005, the specification of which is incorporated herein by reference in its entirety. The service center server 134 may forward e-mail alerts to service personnel in response to receiving maintenance requests to inform the service personnel that a particular machine at a particular location requires servicing. Also, the service center server 134 may be configured to communicate software upgrades or software patches to the exercise machines 120 and 122, the servers 112, the PDA 128, the kiosks 118a-c, etc.

The databases or data structures used by the server 134 and the servers 112 may be implemented using relational databases to store, among other things, user (e.g., fitness user, rehabilitation patient, etc.) demographic information, exercise and/or rehabilitation programs in which users are enrolled, workout schedules, results of previous workout sessions, etc. Other information that may be stored within the databases includes: a list of all fitness equipment in the fitness system, association of network devices to fitness devices, class schedules, upcoming outdoor activities, etc.

The servers 134 and 112 may also be used to perform data analyses. For example, a fitness center manager can perform ad-hoc queries to determine the popularity of fitness programs and equipment. Also, a physical therapist can perform ad-hoc queries to determine the percentage of patients that are meeting their rehabilitation goals, the percentage of patients that are in compliance with a rehabilitation program, etc.

FIG. 2 depicts a plurality of example entities that may be communicatively coupled to the example fitness network system 100 (FIG. 1) to exchange fitness information with the data center 104a (FIG. 1). In the illustrated example, a medical facility 202, an insurance company 204, a health maintenance organization (HMO) 206, and a home 208 may be communicatively coupled to the data center 104a via the communication network 108. The medical facility 202 may be a physician's office, a physical therapy or rehabilitation center, a hospital, etc. that provides medical professionals access to fitness information of patients/fitness members that is stored in the data center 104a. In this manner, medical professionals may update or supplement the fitness information with health status information and may monitor physical fitness progress, therapy progress, or physiological status (e.g., blood pressure) of patients/fitness members. Medical professionals may update workout programs, workout schedules, and/or exercise machine programs for patients having fitness information stored in the data center 104a. For example, the medical professionals may create or update exercise machine programs to enable patients to exercise in a manner consistent with their health conditions. The medical professionals may also exchange messages (e.g., text messages) with fitness members via the fitness network system 100.

The insurance company 204 and the HMO 206 may base insurance rates on various health criteria. To reduce overall healthcare costs, the insurance company 204 and the HMO 206 may implement incentive programs that lower insurance rates for people who exercise regularly. To accurately assess a person's health status, the insurance company 204 and the HMO 206 may access the fitness information stored at the data center 104a. Also, the insurance company 204 and the HMO 206 can monitor fitness members' frequency of exercise to determine if the fitness members qualify for reduced insurance rates under particular incentive programs. Additionally, the insurance company 204 and the HMO 206 may use the fitness information to approve benefits covering, for example, a rehab program based on the fitness information. The insurance company 204 and the HMO 206 may also modify or update the fitness information to include, for example, approval information. In this manner, a physician, a physical therapist, etc. may access the approval information to determine whether to proceed with particular treatments.

Fitness members may access their fitness information when exercising at home (e.g., the home 208). In the illustrated example, the home 208 includes a wireless network access point 210, an exercise machine 212, and a network device 214 that communicatively couples the exercise machine 212 to the communication network 108 via the wireless network access point 210. The wireless network access point 210 may be substantially similar or identical to the wireless network access point 114 (FIG. 1), and the network device 214 may be substantially similar or identical to the network devices 116 (FIG. 1). A fitness member may access fitness goals, workout schedule, exercise machine programs, etc. from the data center 104a to exercise at home. The exercise machine 212 also acquires fitness progress and physiological measurements at the home 208, and communicates the information to the data center 104a. Additionally or alternatively, physiological sensors may be communicatively coupled to the wireless network access point 210 and may communicate physiological measurements to the data center 104a independent of the exercise machine 212. In this manner, the fitness information for any member is always updated whether the fitness member exercises at home, at the fitness facility 102, or at any other fitness facility. The home 208 also includes a home computer 216 that is substantially similar or identical to the remote computer 106 and/or the server 112 of FIG. 1. The computer 216 may process workout requests and/or results associated with the home 208, thus, allowing a fitness system located in the home 208 to operate during times when network access from the home 208 is unavailable for communicating with the data center 104a. When communication is reestablished, the computer 216 may replicate and/or exchange fitness data with the data center 104a using, for example, synchronization logic.

In some cases, health care professionals or personal trainers visit the home 208 to assist or monitor a fitness member during exercise sessions. In the illustrated example, a home health care professional 218 is shown carrying a wireless-enabled portable computing device 220 that is communicatively coupled to the communication network 108 via the access point 210. The health care professional 218 may use the portable computing device 220 to access fitness information stored at the data center 104a while visiting the home 208. Application programming interfaces (API's) used to implement web-based applications for exchanging the fitness information between medical professionals, insurance companies, HMO, home terminals, or other third parties and the data center 104a may exchange the fitness information in a manner that complies with the Health Insurance Portability and Accountability Act (HIPPA) or other privacy regulations.

FIG. 3 is a detailed block diagram of an example exercise machine control system 300 (i.e., an exercise machine data terminal 300 or an information device 300) that may be implemented in connection with an example exercise machine 302 to implement at least some of the example methods and systems described herein. Although the example exercise machine 302 is shown as a treadmill, the control system 300 may be implemented in connection with any type of exercise machine such as, for example, weight machines, elliptical trainer machines, stepper machines, stationary bicycles, etc. The control system 300 is configured to control operations of the exercise machine 302, monitor functionality and usage of the exercise machine 302, and monitor a person's physiological status. The control system 300 is also a network node that is communicatively coupled to other network nodes (e.g., the data centers 104a-b of FIG. 1, the local facility backend server 130 of FIG. 1, the service center 132 of FIG. 1, etc.) via the communication network 108, and configured to exchange fitness information and any other information (e.g., exercise machine programs, maintenance information, etc.) with the other network nodes.

The structures shown in FIG. 3 may be implemented using any desired combination of hardware and/or software. For example, one or more integrated circuits, discrete semiconductor components, or passive electronic components may be used. Additionally or alternatively, some or all, or parts thereof, of the structures of FIG. 3 may be implemented using instructions, code, or other software and/or firmware, etc. stored on a computer-readable medium that, when executed by, for example, a processor system (e.g., the processor system 810 of FIG. 8), perform at least some of the methods disclosed herein. Of course, the structures of FIG. 3 are shown and described below by way of example, and any portion or portions thereof may be changed or rearranged to produce results similar or identical to those disclosed herein.

As shown in FIG. 3, the control system 300 includes a system controller 304 which is used to interface and exchange information with the peripheral devices of the control system 300 as described below. The system controller 304 may be configured to store and retrieve information and to decode and execute machine readable instructions and perform operations based on those instructions. The system controller 304 may also be configured to process and analyze data such as, for example, physiological measurement data, machine monitoring and usage data, network data, etc. The system controller 304 may be implemented using any suitable processor and/or dedicated hardware.

The control system 300 includes a network interface device 306 (i.e., a data communication interface 306) that is communicatively coupled to the system controller 304 and the communication network 108 and which enables the system controller 304 to exchange information with other network nodes via the communication network 108. The network interface device 306 may be implemented using any wired or wireless network hardware and protocol including, for example, Ethernet, IEEE 1394, Bluetooth®, IEEE 802.11, etc. Also, the network interface device 306 may be used to implement any of the network devices 116 of FIG. 1 and may be communicatively coupled to the communication network 108 via the wireless network access point 114 of FIG. 1.

The control system 300 includes a physiological sensors interface 308 that is configured to be wired or wirelessly communicatively coupled to one or more physiological or biometric sensors. For example, the physiological sensors interface 308 may be communicatively coupled to handrail or handgrip sensors 310 configured to measure the heart rate of a person 312 (e.g., a fitness member) when the person's hands are placed on the sensors 310. The physiological sensors interface 308 may also be wirelessly communicatively coupled to one or more wearable physiological sensors or biometric sensors. For example, as shown in FIG. 3, the person 312 is wearing a wireless-enabled wrist sensor 314 to monitor or measure the heart rate of the person 312. The person 312 is also wearing a chest strap sensor 316 configured to measure heart rate. Each of the sensors 314 and 316 is configured to communicate heart rate signals or information to the physiological sensors interface 308. Additionally or alternatively, the sensors 314 and 316 may be configured to wirelessly communicatively couple to a wireless network access point (e.g., the wireless network access points 114 and 210) and communicate physiological information directly to one or more servers (e.g., the servers 112 and/or the local backend server 130) via the network 108.

The physiological sensors interface 308 may communicate the physiological measured information to the system controller 304. The system controller 304 may then process and/or analyze the physiological measured information. For example, the system controller 304 may compare the physiological measured information to physiological target information to determine whether the person 312 is exercising within a predetermined range (e.g., a target heart rate range). The system controller 304 may then communicate the processed physiological information to the data centers 104a-b (FIG. 1) via the communication network 108 for storage within fitness information records of the person 312. Alternatively or additionally, the system controller 304 may communicate the raw physiological measurements to the data centers 104a-b and the servers 112 may process and/or analyze the information.

The control system 300 includes a machine sensors interface 318 configured to obtain measurement information associated with operations of the exercise machine 302 associated with user settings (e.g., speed, incline, etc.) and machine operating conditions (e.g., motor heat, machine vibration, etc.). In an example embodiment, the machine sensors interface 318 is communicatively coupled to a speedometer (not shown) and is configured to obtain speed information indicative of how fast the treadmill belt is moving. The machine sensors interface 318 may also be communicatively coupled to other machine sensors such as, for example, an incline sensor, a vibration sensor, a temperature sensor, etc. The machine sensors interface 318 communicates machine measurement information to the system controller 304.

The system controller 304 may process and analyze the machine measurement information and perform operations based on the analyses and/or communicate the process machine measurement information to the data centers 104a-b. For example, the system controller 304 may monitor the operating conditions of each subsystem of the exercise machine 302 to ensure that they are working properly. The system controller 304 may generate a maintenance ticket if it determines that any machine subsystem is not working properly and communicate the maintenance ticket to the service center 132 (FIG. 1).

The system controller 304 may communicate speed and incline information to the data centers 104a-b for storage in fitness information data records associated with the person 312. The system controller 304 may also use the machine measurement information to generate and store statistical usage information (e.g., maximum, average, minimum speeds, peak usage times, etc.) associated with fitness members that use the exercise machine, and communicate the statistical usage information to the data centers 104a-b.

The control system 300 includes an input interface 320 and a machine controls interface 322, each of which is communicatively coupled to the system controller 304. The input interface 320 may be implemented using any type of user input device including, for example, buttons, pads, a touchscreen, etc. The input interface 320 enables a user to enter user information (e.g., member ID), machine settings information (e.g., speed, incline, etc.), requests to retrieve exercise programs or fitness information (e.g., a user's physiological target information), etc. For example, a user may request a particular exercise machine program. In this case, the system controller 304 may send a request via the communication network 108 to the data centers 104a-b or to the local facility backend server 130 (FIG. 1). The machine controls interface 322 may then control the speed settings and incline settings of the exercise machine 302 based on the exercise program by communicating control signals to subsystems of the exercise machine 302 such as, for example, a motor (not shown), an incline device (not shown), etc.

The control system 300 includes a memory 324, which is used to store any information associated with the exercise machine 302 and the person 312. For example, the memory 324 may be used to store exercise machine programs downloaded for operation of the exercise machine 302. The memory 324 may also be used to store usage information and maintenance information associated with the exercise machine 302 and/or fitness information associated with the person 312. The memory 324 may be implemented using any suitable volatile or non-volatile memory.

The control system 300 also includes a clock 326 that is communicatively coupled to the system controller 304 and configured to maintain a current time of day. The clock 326 may be synchronized with clocks associated with other network nodes (e.g., the local facility backend server 130, the servers 112 of the data centers 104a-b of FIG. 1). The clock 326 may be used to determine when a fitness member should move to a next exercise machine, program, or activity based on the fitness member's workout schedule retrieved from the data centers 104a-b.

The control system 300 also includes an RF sensor 328 that is communicatively coupled to the system controller 304 and configured to detect RFID device's worn by fitness members. In the illustrated example, the RF sensor 328 is configured to detect a member ID associated with the person 312 and stored in an RFID device 330 worn by the person 312. In this manner, when the person 312 requests use of the exercise machine 302, the system controller 304 may send the member ID of the person 312 to the data centers 104a-b and/or the local facility backend server 130 to retrieve fitness information associated with the person 312. For example, the system controller 304 may obtain exercise goals, exercise target data, workout schedules, etc. based on the member ID. In one example embodiment, fitness members may have specific privilege levels to use particular exercise equipment and/or only some functions of exercise machines based on their membership level status. In this case, the system controller 302 may obtain privilege levels based on the member ID's.

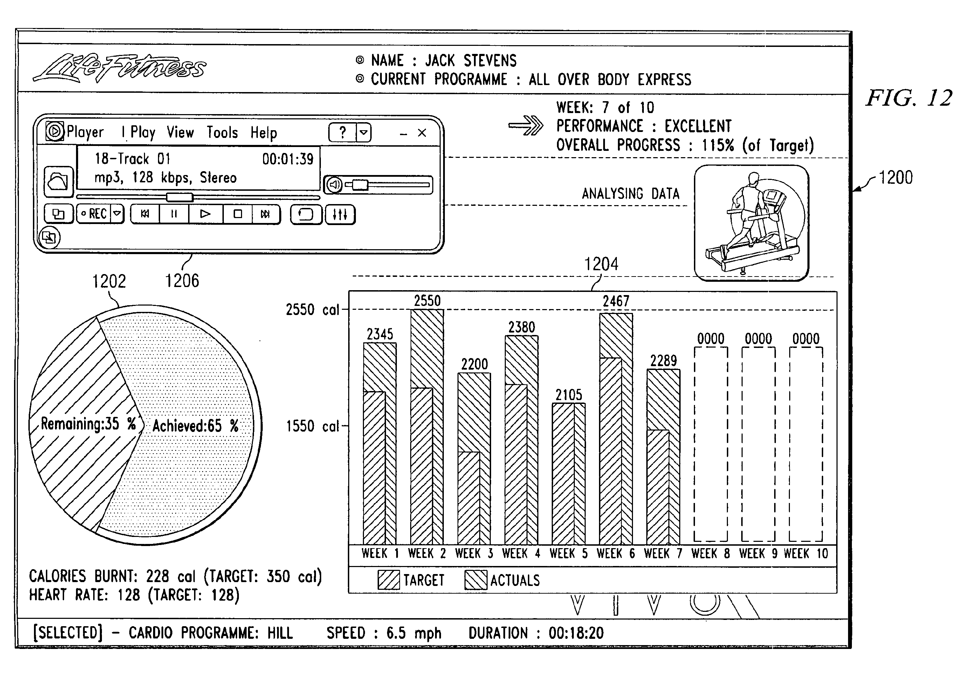

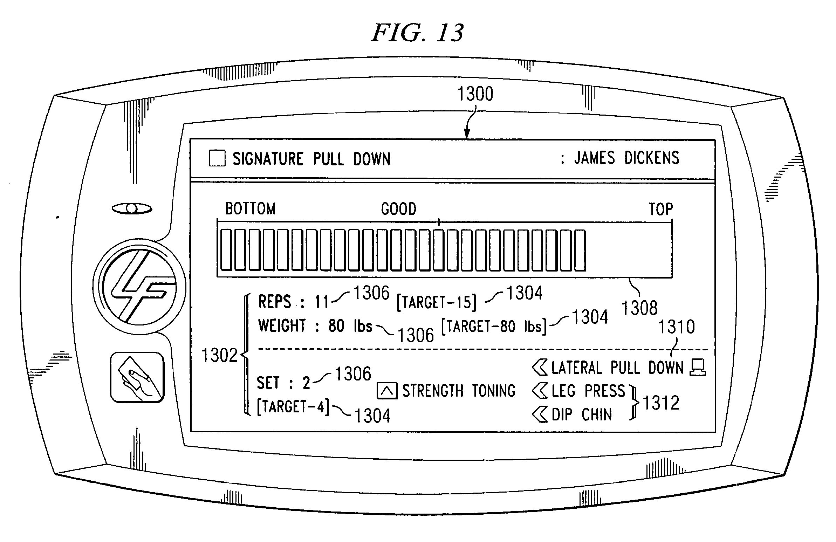

The control system 300 includes an output interface 332 that is communicatively coupled to the system controller 304 and configured to display visual information (e.g., exercise machine console user interfaces 1200 and 1300 of FIGS. 12 and 13) or emit audio alerts. The information may be fitness information (e.g., workout goals, exercise machine programs, etc.), exercise machine operating information (e.g., speed, incline, etc.), notifications, or any other information. In one example implementation, the output interface 332 may display message waiting notifications to alert the person 312 when a new message has been received in the person's message inbox. In this manner, the person 312 is alerted to check messages at the kiosk terminal 118b when finished using the exercise machine 302. The output interface 332 may also be configured to display a class starting notification when an exercise class of interest to the person 312 is about to start.

The output interface 332 may also be configured to display an alert when the system controller 304 determines based on the person's workout schedule that the person 312 should move on to a next exercise indicated in the workout schedule. For example, the system controller 304 may identify a subsequent exercise machine in a person's exercise routine based on a workout schedule and an ID of the current machine (e.g., the exercise machine 302) and cause the output interface 332 to display the name of the subsequent exercise machine on which the person 312 should exercise. Of course, the output interface 332 may be used to output any other type of information. The output interface 332 may be implemented using any type of display technology well known in the art including, for example, a liquid crystal display (LCD), a plasma display, a light emitting diode (LED), a cathode ray tube (CRT), etc. Additionally, the output interface 332 may include a speaker or other audio emitting device.

In an example implementation, the control system 300 may be configured to provide guidance information to fitness members based on fitness member behavior. For example, the control system 300 may issue warnings and/or advisements to the person 312 via the output interface 332 if the control system 300 determines the person 312 is endangering physical wellness by, for example, starting to run at a relatively fast speed before warming up with a slow jog. Additionally, a weight training machine (e.g., the exercise machines 120 of FIG. 1) may display a warning and/or advisement to a fitness member if the weight training machine determines that the fitness member has not previously warmed up using, for example, a treadmill or other cardio equipment, prior to using the weight training machine. Specifically, the backend server 130 may continuously log a fitness member's activities within the fitness facility 102. The control system 300 may retrieve the activity log information to determine what types of activities the fitness member has previously performed. If the control system 300 is associated with a weight training machine and determines based on the activity log information that the fitness member has not previously warmed up, then the control system 300 can provide guidance information (e.g., a warning and/or advisement) to the fitness member via the output interface 332 indicating that the fitness member can avoid injury by first warming up using, for example, a treadmill, prior to using the weight training machine.

The control system 300 may also be configured to display a fitness member's progress over a particular length of time. Specifically, the control system 300 may retrieve fitness progress information for a fitness member (e.g., the person 312) from the backend server 130 and/or the servers 112 and display the fitness progress information via the output interface 332 (e.g., via a current progress indicator chart 1202 and/or a historical progress indicator chart 1204 of FIG. 12). In an example implementation, the control system 300 may cause the output interface 332 to display the target fitness information (e.g., target miles run, target heart rate, target weight plates, etc.) adjacent to measured fitness information (e.g., actual miles run, measured heart rate, current weight plate setting, etc.). The target and measured fitness information may be displayed based on temporal information such as, for example, a current day (e.g., day 5 of a 30 day program), a current week (e.g., week 2 of a 6 week program), or any other time so that fitness members may observe their progress as they exercise.

Additionally, the control system 300 may automatically adjust or control settings (e.g., speed, incline, resistance, etc.) of the exercise machine 302 based on the target fitness information. For example, the control system 300 may enable the person 312 to interval train by automatically adjusting speed or incline settings of the exercise machine 302 via the machine controls interface 322 as the person 312 exercises (e.g., runs) based on the target fitness information and physiological measurement information obtained via the physiological sensors interface 308. In this manner, instead of entering a plurality of information (e.g., target speed, target incline, target heart rate, etc.) via the input interface 320 prior to using the exercise machine 302, the person 312 need only provide a fitness member ID to the control system 300 so that the control system 300 can retrieve from the backend server 130 and/or the servers 112 the target fitness information associated with the person 312 that enables the control system 300 to automatically adjust the settings of the exercise machine. Also, the system controller 304 may cause the output interface 332 to display a graph or other visual indicator indicating the settings of the exercise machine 302 as they are adjusted.

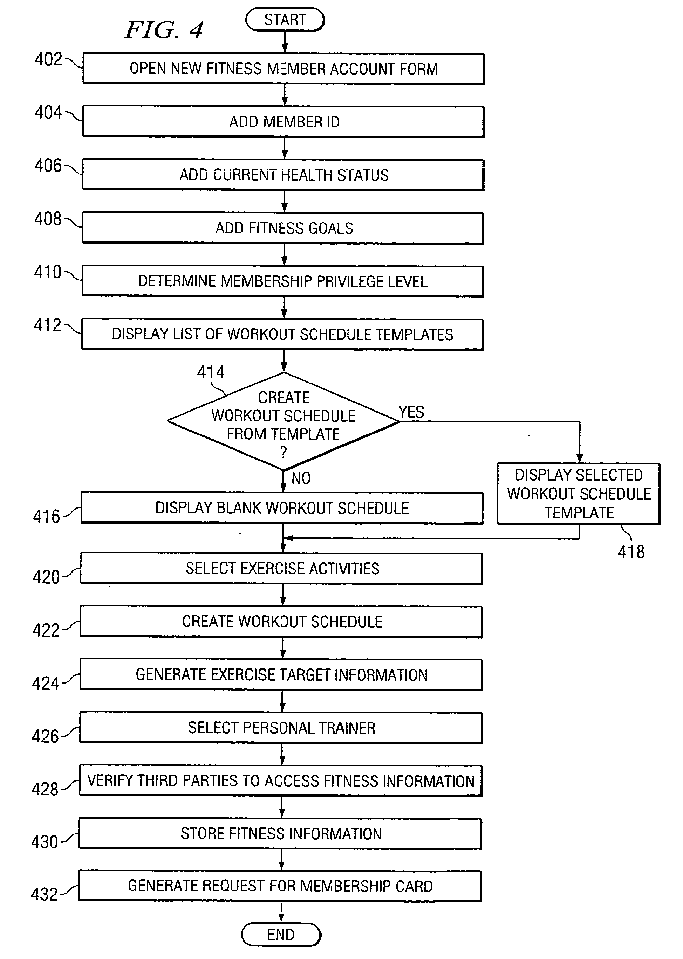

FIGS. 4, 5A, 5B, 6, and 7 illustrate flowcharts that depict example methods that may be used to implement the example fitness network system 100 of FIG. 1. The example methods depicted in the flow diagrams of FIGS. 4, 5A, 5B, 6, and 7 may be implemented in software, hardware, and/or any combination thereof. For example, the example methods may be implemented in software that is executed via the example control system 300 of FIG. 3, the example processor system 810 of FIG. 8, and/or a hardware system configured according to the example control system 300. Although, the example methods are described below as a particular sequence of operations, one or more operations may be rearranged, added, and/or eliminated to achieve the same or similar results.

FIG. 4 is a flowchart of an example method that may be used to store fitness information in a data record associated with a fitness member account. A fitness member, a personal trainer, or any other person (e.g., a physical trainer, a physician, fitness facility personnel, etc.) may create the fitness member account using any network node (e.g., the kiosk terminal 118b, the fitness administrator terminal 118c, the remote terminal 106, the wireless-enabled portable computing device 128, etc. of FIG. 1) that is communicatively coupled to the fitness network system 100 via the communication network 108 (FIG. 1). The fitness member account may be stored in one or more databases stored in various servers of the fitness network system 100. For example, the database may be stored in the servers 112 of the data centers 104a-b and/or the local facility backend server 130 of FIG. 1. In this manner, the fitness information may be retrieved from the fitness facility 102 (FIG. 1) or from any other fitness facility by fitness members and/or any third party entity (e.g., the medical facility 202, the insurance company 204, the HMO 206, the home 208 of FIG. 2, etc.).

Initially, a person opens a new fitness member account form (block 402). For example, the person may select to open a new fitness member account template having predefined fields for various fitness information, where each field is associated with a data field entry in a database record. The person then adds a member ID (block 404) of the fitness member for which the new fitness member account is being created. The person then adds the current health status of the fitness member (block 406). For example, the person may add height, weight, percent body fat, ailments, allergies, adverse or any other health conditions, etc. The person then adds the fitness goals of the fitness member (block 408). The fitness goals may include, cardiovascular goals, strength goals, weight goals, body fat percent goals, etc.

The computer terminal at which the new fitness member account is being created then obtains the membership privilege level (block 410) associated with the membership ID and displays a list of workout schedule templates (block 412). The computer terminal may obtain the membership privilege level from, for example, the fitness administrator terminal 118c, the facility local backend server 130, or the data centers 104a-b. The membership privilege level may be based on the price paid for the membership. The computer terminal may display the workout schedule templates based on the membership privilege level determined at block 410 and the fitness goals provided at block 408. For example, if the person entered strength training fitness goals, then the computer terminal may display workout schedule templates with an emphasis on weight training. If the membership privilege level includes access to, for example, racquetball courts or studio classes, then the computer terminal may display workout schedule templates having those types of exercise activities. The computer terminal then determines whether the person would like to create a workout schedule based on one of the workout schedule templates displayed at block 412 (block 414). For example, if the person selects one of the workout schedule templates, then the computer terminal determines that the person would like to use a workout schedule template to create a workout schedule. However, if the person does not select one of the workout schedule templates, but instead selects a blank schedule form, then the computer terminal determines that the person would like to create a workout schedule from a blank schedule form. If the computer terminal determines that the person would not like to use one of the workout schedule templates, then the computer terminal displays a blank workout schedule form (block 416).

If the computer terminal determines that the person would like to use one of the workout schedule templates, then the computer terminal displays the selected workout schedule template (block 418). The selected workout schedule template may include one or more workout program templates directed to particular fitness goals (e.g., physical therapy, rehabilitation, strength training, cardiovascular training, sports training, weight loss, etc.). The workout program templates may include a list of exercises or activities and limits or thresholds (e.g., a running speed limit, a weight resistance limit, a repetition limit, etc.) associated with each exercise based on a person's physical condition or health. For example, the limits or thresholds for a physical therapy workout program template may be directed to limiting the amount of resistance or stress a person exerts using an injured muscle, bone, etc.

After a blank workout schedule form or a workout schedule template is displayed at block 416 or block 418, the person selects exercise activities (block 420). The exercise activities may be selected from the template or from another source such as, for example, a list of exercise activities, user-provided exercise activities, etc. The computer terminal then creates the workout schedule based on the user-selected exercise activities (block 422). Then the computer terminal generates exercise target information (block 424) based on the user-selected exercise activities provided at block 420 and the fitness goals provided at block 408. For example, the computer terminal may generate physiological target goals such as, for example, heart rate, for use in connection with cardiovascular exercises (e.g., treadmill workouts). Additionally, for each weight training exercise the computer terminal may generate weight training target information such as, for example, amount of weight, number of sets, number of repetitions, etc.

The person then selects a personal trainer (block 426). For example, the person may select from a list of personal trainers associated with the fitness facility 102 (FIG. 1). Alternatively, the person may enter information about a private personal trainer not listed or not employed by the fitness facility 102. The person then verifies the third parties that should have access to the fitness information (block 428) of the fitness member for which the member account is being created. The person may select from a list of affiliated third parties such as, for example, the medical office 202, the insurance company 204, or the HMO 206 of FIG. 2. The person may also enter information about a third party not listed by the computer terminal.

The computer terminal then stores the fitness information (block 430). For example, the computer terminal may store the fitness information in the servers 112 of the data centers 104a-b and/or the local facility backend server 130 of FIG. 1. The computer terminal can then create a request for a membership card (block 432). For example, the computer terminal may send a message with the member ID to the fitness administrator terminal 118c of the fitness facility 102 requesting creation of a membership card for the indicated member ID. The membership card may include an RFID chip or an RFID device that is encoded with the member ID and that is readable by the RFID device sensors 124 located throughout the fitness facility 102.

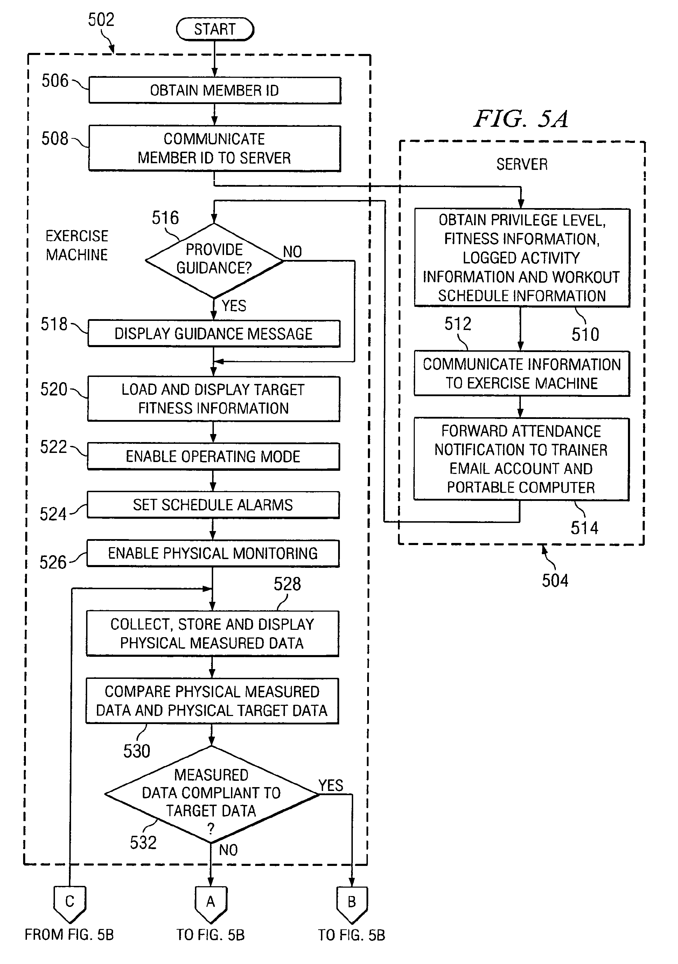

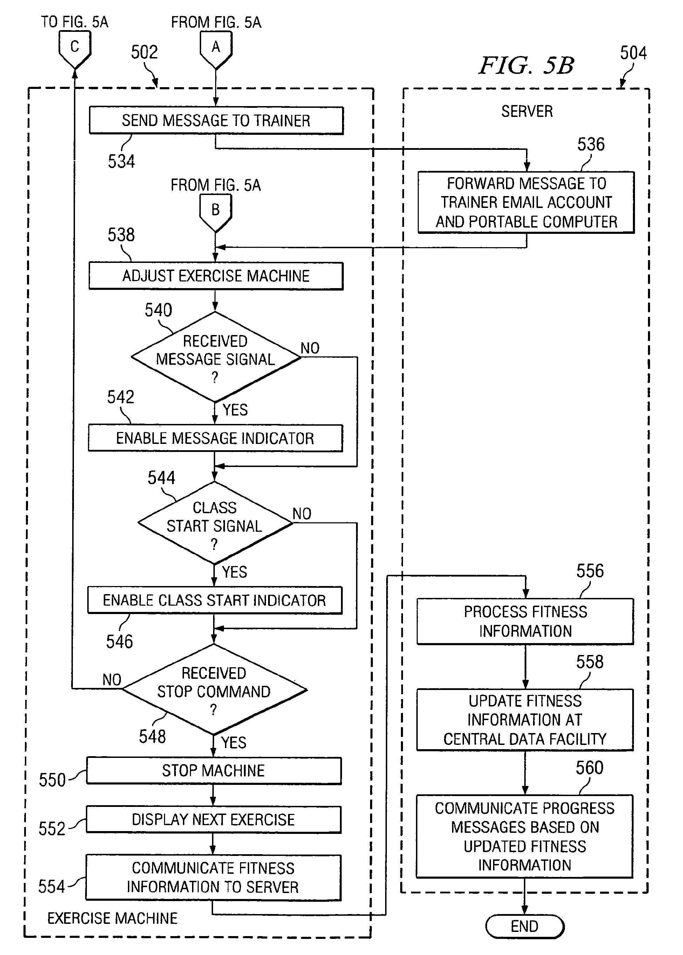

FIGS. 5A and 5B depict a flowchart of an example method that may be used to implement operations of an exercise machine and a server that are communicatively coupled to the example fitness network system 100 of FIG. 1. The flow chart of FIG. 5 includes a first plurality of operations 502 performed by a control system (e.g., the control system 300 of FIG. 3) of an exercise machine, and a second plurality of operations 504 performed by a server (e.g., the local facility backend server 130 or the servers 112 of the data centers 104a-b of FIG. 1) of the fitness network system 100. Although the example method of FIG. 5 is described below with respect to the control system 300 of FIG. 3 and the local facility backend server 130 of FIG. 1, the example method may be implemented using any other suitable server (e.g., the servers 112 in the data centers 104a-b of FIG. 1) or exercise machine control system.

Initially, the system controller 304 obtains the member ID (block 506) via, for example, the RF sensor 328. For instance, the RF sensor 328 may detect the member ID from the RFID device 330 worn by the person 312 using the exercise machine 302 of FIG. 3. The system controller 304 then communicates the member ID to the backend server 130 (block 508) via, for example, the network interface 306 (FIG. 3). The backend server 130 then obtains a privilege level, fitness information, and a member workout schedule (block 510) based on the member ID. For example, the backend server 130 may retrieve the information from local storage or from one of the servers 112 of the data centers 104a-b via the communication network 108. In either case, the backend server 130 then communicates the privilege level, the fitness information, and the workout schedule to the exercise machine (block 512). The backend server 130 also communicates an attendance notification to an e-mail account and the wireless-enabled portable computing device 128 (FIG. 1) of the personal trainer 126 (FIG. 1) (block 514).

The system controller 304 then determines if it should provide guidance to the person 312 (block 516). For example, the system controller 304 may analyze the logged activity information provided by the backend server 130 at block 512 to determine if using the exercise machine 302 endangers the physical wellness of the person 312 (e.g., may cause injury to the person 312) or is otherwise not recommended based on fitness activities previously performed by the person 312. If the system controller 304 determines that it should provide guidance to the person 312, then the system controller 304 displays a warning and/or advisement message (block 518) via the output interface 332 (FIG. 3).

The system controller 304 then loads and displays the target fitness information (block 520) associated with the person 312. For example, the system controller 304 may extract the target fitness information including the physiological target data generated at block 424 of FIG. 4 from the fitness information obtained from the backend server 130. The physiological target data may include a target heart rate, target calories burned, a target running distance, etc. The system controller 304 then enables the operating mode of the exercise machine 302 (block 522). The system controller 304 may enable the operating mode based on the privilege level, the fitness information, and the workout schedule obtained from the backend server 130. For example, if the membership privilege level indicates that the person 312 has restricted privileges, then the system controller 304 may enable the exercise machine 302 to operate in a restrictive mode (e.g., a time-limited mode, a mode limiting the exercise machine 302 to a subset of exercise machine programs, a manual mode, etc.). For example, the restrictive mode may enable only partial configurability of the exercise machine 302. A partial configurability mode may allow a fitness member to only use the exercise machine 302 with a subset of exercise machine programs or to only use a subset of features of the exercise machine 302 or to configure the exercise machine 302 to operate for a limited time. Additionally or alternatively, the system controller 304 may enable the operating mode of the exercise machine 302 based on exercise machine programs indicated in the workout schedule and the fitness information.

The system controller 304 then sets workout schedule alarms (block 524) based on the workout schedule obtained from the backend server 130. The schedule alarms are used to emit audio alerts or display visual alerts via, for example, the output interface 332 (FIG. 3) when the person 312 should move on to another workout or attend a studio class based on the workout schedule. The alerts are emitted or displayed based on comparisons of the times in the workout schedule to the current time values obtained from the clock 326 (FIG. 3).

The system controller 304 then enables physiological monitoring (block 526). For example, the system controller 304 may enable the physiological sensors interface 308 (FIG. 3) to communicate with any wired or wireless physiological sensors including, for example, the handrail sensors 310, the wireless wrist sensor 314, and/or the chest strap sensor 316. In this manner, the system controller 304 can obtain physiological measured data of, for example, the heart rate of the person 312. The system controller 304 then collects, stores, and displays physiological measured data (block 528). For example, the system controller 304 can obtain the physiological measured data from the physiological sensors interface 308, store the data in the memory 324, and display the data via the output interface 332. At block 528 the system controller 304 may also display progress information by displaying a person's long-term goals or workout program goals adjacent to the person's current progress (e.g., cumulative miles at week 8 of a 12 week marathon training program).

The system controller 304 then compares the physiological measured data obtained at block 528 to the physiological target data loaded at block 520 (block 530), and determine if the physiological measured data is compliant to the physiological target data (block 532). For example, the physiological target data may include maximum and minimum threshold values that the system controller 304 compares to the physiological measured data to determine whether the person 312 is exercising within a particular target cardiovascular zone. If the system controller 304 determines at block 532 that the physiological measured data is not compliant to the physiological target data, then the system controller 304 sends a message to the personal trainer (e.g., the personal trainer 126 of FIG. 1) of the person 312 (block 534). The message may include information associated with the non-compliant physiological measured data, and may also include some or all of the physiological measured data and/or the physiological target data. The message is communicated to the backend server 130, and the backend server 130 forwards the message to the personal trainer's e-mail account and to the wireless-enabled portable computing device 128 (FIG. 1) of the personal trainer 126 (block 536).

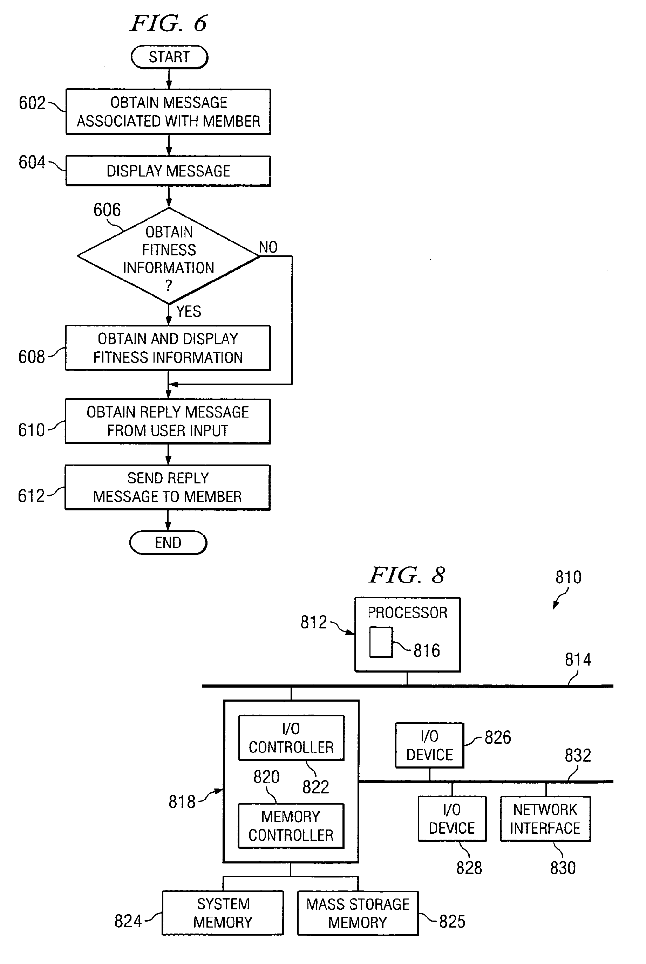

If the system controller 304 determines at block 532 that the measured data is compliant to the target data or after the system control 304 sends the message to the trainer at block 534, the system controller 304 adjusts the settings (e.g., speed, incline, resistance, etc.) of the exercise machine 302 (block 538). For example, the system controller 304 may adjust the exercise machine settings based on the physiological measured data and the physiological target data. Alternatively or additionally, the system controller 304 may adjust the exercise machine settings based on progress information and temporal information (e.g., week 8 of 12) associated with a fitness program. The system controller 304 then determines if it has received a message signal (block 540). A message signal may be sent to the system controller 304 by the backend server 130 or the servers 112 to indicate that an e-mail account or message inbox of the person 312 has received a message. The message may be sent by the trainer 126 in response to the message sent to the trainer 126 at block 534. The example method of FIG. 6 depicts an example method by which the personal trainer 126 may receive and reply to messages such as the message described at block 534. Of course, a message associated with the message signal at block 540 may be any other type of message including, for example, an advertisement to shop at the fitness facility shop, a special event advertisement, a facility informational bulletin, or any other type of message sent by the local facility backend server 130. If the system controller 304 determines at block 534 that it has received a message signal, then the system controller 304 enables a message indicator on the output interface 332 (FIG. 3) (block 542). The message indicator may be a visual or audio indicator to inform the person 312 to check an e-mail account or message inbox for new messages.

After enabling the message indicator at block 542 or, if at block 540 the system controller 304 determines that a message signal was not received, the control system then determines if a class start signal has been asserted (block 544). The class start signal may be an interrupt asserted by the system controller 304 based on the schedule alarms set at block 524 and the output value of the clock 326. The class start signal may also be a message broadcast by the backend server 130 indicating that a studio class is about to start. In either case, if the system controller 304 determines that it has received a class start signal, the system controller 304 enables a class start indicator (block 546). The class start indicator may be a visual or audio indicator output via the output interface 332 to inform the person 312 that a class is about to begin.

After the system controller 304 enables a class start indicator at block 546 or if the system controller 304 determines at block 544 that it has not received a class start signal, the system controller 304 then determines if it has received a stop command (block 548). The stop command is received in response to the person 312 pressing a stop button on the exercise machine 302 or in response to the end of an exercise machine program. If the system controller 304 determines that it has not received a stop command, control is passed back to block 528. However, if the system controller 304 has received a stop command, then the system controller 304 stops the exercise machine 302 (block 550) (FIG. 5B) and displays a next exercise (block 552). For example, the system controller 304 may use the workout schedule provided by the backend server 130 at block 512 to determine the next exercise that the person 312 should perform. For instance, the system controller 304 may retrieve from the workout schedule a name of an exercise machine and display the exercise machine name via the output interface 332 (FIG. 3). The system controller 304 may then communicate fitness information to the backend server 130 (block 554). The fitness information may include the physiological measured data, running distance, calories burned, progress information, etc.

The backend server 130 then processes the fitness information (block 556). For example, the backend server 130 may determine maximum, minimum, average values for various data (e.g., speed, incline, etc.), or may determine if the person 312 was exercising within a predetermined target zone. The backend server 130 then updates the fitness information of the person 312 at the central data facilities 104a-b (block 558). For example, the backend server 130 may update database records associated with the person 312 based on the member ID of the person obtained at block 506. The backend server 130 may also communicate progress messages based on the updated fitness information (block 560). For example, if updated fitness information indicates that the person 312 has met some fitness goals, the backend server 130 can send the person 312 congratulatory motivational messages. Or, if the person did not meet some fitness goals, then the backend server 130 can send motivational messages to encourage the person 312 to meet the fitness goals. Each motivational message may include a level of motivation associated with the person 312. Additionally or alternatively, the backend server 130 can send progress messages to personal trainers or fitness facility personnel regarding the progress of the person 312 and/or the motivation level of the person 312. Specifically, the backend server 130 may determine the level of motivation of the person 312 based on the amount of time required by the person 312 to meet fitness goals. If the person does not meet fitness goals within a predetermined amount of time, the backend server 130 may inform a personal trainer or fitness facility personnel that the person 312 is lacking motivation. The process of FIGS. 5A and 5B is then ended.

FIG. 6 is a flowchart of an example method that may be used to implement a fitness-related messaging application on a portable computing device (e.g., the wireless-enabled portable computing device 128 of FIG. 1) that is communicatively coupled to the fitness network system 100 of FIG. 1. The example method of FIG. 6 may, for example, be used to display the message forwarded to the portable computing device 128 at block 536 of FIG. 5A and to send a message from the personal trainer 126 to an inbox of a fitness member (e.g., the person 312 of FIG. 3). Although the example method of FIG. 6 is describe relative to the portable computing device 128, the example method may be implemented using any other computing device including, for example, the kiosk terminal 118b, the fitness administrator terminal 118c, or the remote terminal 106 of FIG. 1.

Initially, the portable computing device 128 obtains a message associated with a fitness member (e.g., the person 312) (block 602). For example, the portable computing device 128 obtains the message sent by the control system 300 via the backend server 130 at blocks 534 and 536 of FIG. 5A. The portable computing device 128 then displays the message via a display (block 604). The portable computing device 128 then determines if it should retrieve fitness information (block 606). For example, after reading the message displayed at block 604, the personal trainer 126 may want to view some of the fitness information such as, for example, member profile information, member workout schedule information, fitness goals, etc. In this manner, the personal trainer 126 may reply to the fitness member with information pertinent to the fitness member's fitness profile. If the portable computing device 128 determines that it should retrieve fitness information, then the portable computing device 128 obtains and displays the fitness information (block 608). For instance, the portable computing device 128 may obtain the fitness information from, for example, the servers 112 or the local facility backend server 130 via the communication network 108.

After the portable computing device 128 obtains and displays the fitness information at block 608, or if the portable computing device 128 determines at block 606 that it should not obtain the fitness information, then the portable computing device 128 obtains a reply message based on user input (block 610) from the trainer. The reply message may include a motivational message, a change to a workout schedule, or any other fitness-related message or information. The portable computing device 128 then communicates the reply message to the fitness member (block 612). For example, the portable computing device 128 may communicate the reply message to the e-mail account or message inbox of the person 312. The process of the example method then ends.

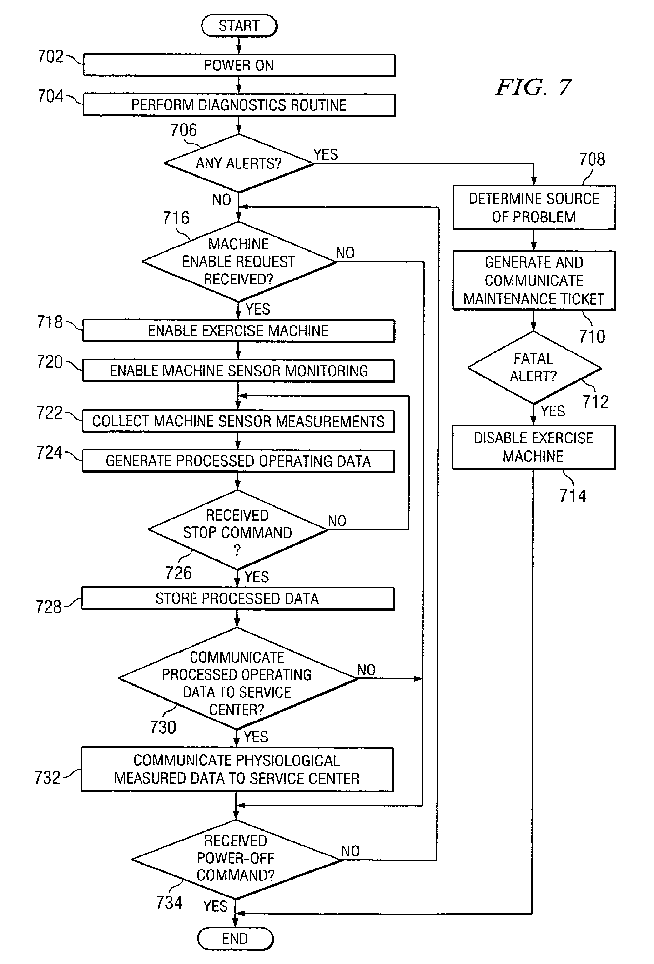

FIG. 7 is a flowchart of an example method that may be used to monitor the operating status of an exercise machine (e.g., the exercise machine 302) that is communicatively coupled to the example fitness network system 100 of FIG. 1. Initially, the exercise machine 302 is powered on (block 702). The system controller 304 then performs a diagnostics routine (block 704). The diagnostics routine may be a software and hardware diagnostics routine that determines the operability of each software and hardware (e.g., circuit hardware and mechanical hardware) subsystem of the exercise machine 302. For example, the diagnostics routine may determine that each software and firmware module stored in the memory 324 and/or loaded into the system controller 304 is operational. The diagnostics routine may also check for malfunctions in the electrical circuits of the system controller 300 and malfunctions in any mechanical (e.g., a treadmill belt) or electro-mechanical (e.g., a motor) subsystems of the treadmill.

The system controller 304 then determines if the diagnostics routine at block 704 issued any alerts (block 706). For example, the diagnostics routine may issue an alert for any malfunction or error found in connection with any of the exercise machine subsystems during execution of the diagnostics routine. If the system controller 304 determines at block 706 that an alert has been issued, then the system controller 304 determines the source of the problem (block 708) associated with the alert. The system controller then generates and forwards a maintenance ticket (block 710). For example, the system controller 304 may generate a maintenance ticket requesting service for the problem identified at block 708 and forward the maintenance ticket to the service center 132 (FIG. 1).

The system controller 304 then determines if the alert received at block 706 is a fatal alert (block 712). A fatal alert represents a problem that prohibits operation of the exercise machine, and may include, for example, a broken motor, a broken belt, a fatal software error, a burned fuse, etc. If the system controller 304 determines at block 712 that the alert is a fatal alert, the system controller 304 disables the exercise machine 302 (block 714) and the process is ended.

If the system controller 304 determines at block 706 that no alerts have been received, then the system controller 304 determines if any machine enable requests have been received (block 716). An enable request is received in response to a person pressing a button or other input device of the exercise machine 302 indicating a request to use the exercise machine 302. If an enable request has been received, then the system controller 304 enables the exercise machine (block 718). In some example embodiments, enabling the exercise machine may require a number of operations such as, for example, the operations described above in connection with blocks 506 through 526 of FIG. 5A. The system controller 304 then enables machine sensor monitoring (block 720). For example, the system controller 304 may enable the machine sensors interface 318 and any machine sensors communicatively coupled to the machine sensors interface 318 to acquire machine sensor measurements (e.g., speed, incline, motor temperature, etc.) while the person 312 is using the exercise machine 302.

The system controller 304 then collects the machine sensor measurements (block 722) from the machine sensors interface 318 and generates process operating data (block 724). For example, the system controller 304 may process the collected machine sensor measurements to determine maximum, minimum, and average values, or any other values. In some example embodiments, the process operating data is subsequently analyzed by the system controller 304 or by another processor system (e.g., the servers 112, 130 of FIG. 1) to assess the operating performance of the exercise machine 302.

The system controller 304 then determines if it has received a stop command (block 726). The stop command is received in response to the person 312 pressing a stop button on the exercise machine 302 or in response to the end of an exercise machine program. If the system controller 304 determines that it has not received a stop command, control is passed back to block 722. However, if the system controller 302 has received a stop command, then the system controller 304 stores the process data generated at block 724 (block 728). For example, the system controller 304 may store the processed data locally in the memory 324 (FIG. 3) or in a server such as, for example, the local facility backend server 130 of FIG. 1.

The system controller 304 then determines if it should communicate the processed data to the service center server 134 (FIG. 1) (block 730). For example the system controller 304 may be configured to communicate the processed data to the service center server 134 at predetermined times (e.g., once a day at midnight, once a week on Sunday at midnight, etc.), in response to a request received from the service center server 134, when the memory 324 is full, or when there is an error with the exercise machine 302. If the system controller 304 determines at block 730 that it should communicate the processed data to the service center server 134, the system controller 304 communicates the processed data to the service center server 134 (block 732) via, for example, the network interface 306 (FIG. 3).

After the system controller 304 communicates the processed data to the service center server 134, or if the system controller 304 determines at block 730 that it should not communicate the processed data to the service center server 134, or if the system controller determines at block 716 that it has not received a machine enable request, then the system controller 304 determines if it has received a power-off command (block 734). The power-off command may be received in response to a manual or automatic shutdown of the exercise machine 302. If the system controller 304 determines that it has not received a power-off command, then control is passed back to block 716. However, if the system controller 304 determines that it has received a power-off command, then the process is ended.

FIG. 8 is a block diagram of an example processor system that may be used to implement the systems and methods described herein. As shown in FIG. 8, the processor system 810 includes a processor 812 that is coupled to an interconnection bus 814. The processor 812 includes a register set or register space 816, which is depicted in FIG. 8 as being entirely on-chip, but which could alternatively be located entirely or partially off-chip and directly coupled to the processor 812 via dedicated electrical connections and/or via the interconnection bus 814. The processor 812 may be any suitable processor, processing unit or microprocessor. Although not shown in FIG. 8, the system 810 may be a multi-processor system and, thus, may include one or more additional processors that are identical or similar to the processor 812 and that are communicatively coupled to the interconnection bus 814.

The processor 812 of FIG. 8 is coupled to a chipset 818, which includes a memory controller 820 and an input/output (I/O) controller 822. As is well known, a chipset typically provides I/O and memory management functions as well as a plurality of general purpose and/or special purpose registers, timers, etc. that are accessible or used by one or more processors coupled to the chipset 818. The memory controller 820 performs functions that enable the processor 812 (or processors if there are multiple processors) to access a system memory 824 and a mass storage memory 825.

The system memory 824 may include any desired type of volatile and/or non-volatile memory such as, for example, static random access memory (SRAM), dynamic random access memory (DRAM), flash memory, read-only memory (ROM), etc. The mass storage memory 825 may include any desired type of mass storage device including hard disk drives, optical drives, tape storage devices, etc.

The I/O controller 822 performs functions that enable the processor 812 to communicate with peripheral input/output (I/O) devices 826 and 828 and a network interface 830 via an I/O bus 832. The I/O devices 826 and 828 may be any desired type of I/O device such as, for example, a keyboard, a video display or monitor, a mouse, etc. The network interface 830 may be, for example, an Ethernet device, an asynchronous transfer mode (ATM) device, an 802.11 device, a DSL modem, a cable modem, a cellular modem, etc. that enables the processor system 810 to communicate with another processor system.

While the memory controller 820 and the I/O controller 822 are depicted in FIG. 8 as separate functional blocks within the chipset 818, the functions performed by these blocks may be integrated within a single semiconductor circuit or may be implemented using two or more separate integrated circuits.

FIG. 9 is an example template generator user interface 900 that may be used to generate exercise program templates. The example template generator user interface 900 may be used to generate exercise program templates associated with machine or non-machine-based exercises. In some example implementations, access to the example template generator user interface 900 may be restricted to fitness facility personnel or health professionals and may be accessed via, for example, the fitness administrator terminal 118c (FIG. 1) or by logging into a privileged account (e.g., an administrator account, a fitness facility personnel account, a physical therapist account, a physician account, etc.) via any computer terminal (e.g., the terminals 118a-c, the remote computer 106 (FIG. 1), etc.) or portable computing device (e.g., the portable computing devices 128 (FIG. 1) and 220 (FIG. 2)). Additionally or alternatively, the example template generator user interface 900 may be accessed by any user of the example fitness network system 100 (FIGS. 1 and 2) including, for example, fitness members.

The template generator user interface 900 may be used to generate templates directed toward particular fitness goals (e.g., cardiovascular training, strength training, physical therapy or rehabilitation training, marathon training, weight loss, sports training, etc.). As shown in FIG. 9, the template generator user interface 900 may include a plurality of parameters 902 that enable a user (e.g., a fitness member, a personal trainer, a physical therapist, an administrator, a physician, etc.) to create workout routines or exercise programs based on individual fitness member capabilities. For example, a physical therapist may specify minimum and maximum-type parameters 904 that restrict the amount of physical exertion during particular exercises or that restrict settings for particular exercise machines. The physical therapist may generate a template that restricts the amount of weight resistance that can be set for a leg extension machine if a fitness member is participating in physical therapy sessions for a knee injury. A personal trainer may use the example template generator user interface 900 to generate an example marathon training template that restricts a running duration or the speed at which a fitness member should run on a treadmill during particular days of a marathon training program.

FIGS. 10 and 11 are example template user interfaces 1000 and 1100 depicting exercise program templates associated with exercise machines (e.g., the exercise machines 120 and 122 of FIG. 1 or the exercise machine 302 of FIG. 3). In particular, the example template user interface 1000 is associated with a treadmill exercise machine, and the example template user interface 1100 is associated with a weight training exercise machine. The templates depicted in the example template user interfaces 1000 and 1100 may be generated based on the example template generator user interface 900 of FIG. 9. The example template user interfaces 1000 and 1100 may be accessed by a user (e.g., a fitness member, a personal trainer, a physical therapist, an administrator, a physician, etc.) via any computer terminal (e.g., the terminals 118a-c, the remote computer 106 (FIG. 1), etc.) or portable computing device (e.g., the portable computing devices 128 (FIG. 1) and 220 (FIG. 2)) and used to define a plurality of parameters 1002 and 1102 that are stored in, for example, the servers 112 or the local backend server 130 (FIG. 1). The values associated with the plurality of parameters 1002 and 1102 may be subsequently accessed by a fitness member and/or an exercise machine (e.g., the exercise machines 120 and 122 of FIG. 1 or the exercise machine 302 of FIG. 3) to perform the specified exercises accordingly.

FIGS. 12 and 13 are example exercise machine console user interfaces 1200 and 1300 associated with exercise machines (e.g., the exercise machines 120 and 122 of FIG. 1 and/or the exercise machine 302 of FIG. 3) communicatively coupled to the example fitness network system of FIG. 1. In particular, the example exercise machine console user interface 1200 (i.e., the console user interface 1200) is associated with a treadmill exercise machine, and the example exercise machine console user interface 1300 (i.e., the console user interface 1300) is associated with a weight training exercise machine. The console user interfaces 1200 and 1300 may be displayed via, for example, the output interface 382 (FIG. 3), and configured to display fitness information retrieved from the servers 112 and/or 130 of FIG. 1 (e.g., exercise machine programs, exercise program parameter values, physiological target information, etc.), fitness information measured by an exercise machine (e.g., physiological measured information), and analyses information. As shown in FIG. 12, the console user interface 1200 includes a current progress indicator chart 1202 and a historical progress indicator chart 1204. In the illustrated example, the current progress indicator chart 1202 displays progress information associated with a current exercise session indicating that a fitness member (e.g., the user 312 of FIG. 3) has burnt 65% of a target amount of calories. The historical progress indicator chart 1204 the weekly total amounts of calories burnt for each of the previous weeks. In the illustrated example of FIG. 12, the console user interface 1200 also includes a media player interface 1206 that may be used to access audio media and control audio playback. Audio media may be accessed from the servers 112 and/or 130 of FIG. 1 or from machine accessible mediums (e.g., magnetic and/or optical disks, memory cards, etc.).

As shown in FIG. 13, the console user interface 1300 includes a current status indicator 1302 depicting target information 1304 and measured information 1306 associated with the amount of repetitions performed in a current exercise set and a weight resistance setting for an associated weight training machine. The console user interface 1300 also includes an exercise form indicator 1308 that may be used to indicate whether a fitness member is properly performing an exercise by moving through a full range of motion. Also, the console user interface 1300 includes a current exercise indicator 1310 and a next exercises indicator 1312. The next exercises indicator 1312 displays the names of the next exercises specified in a fitness member's workout schedule.

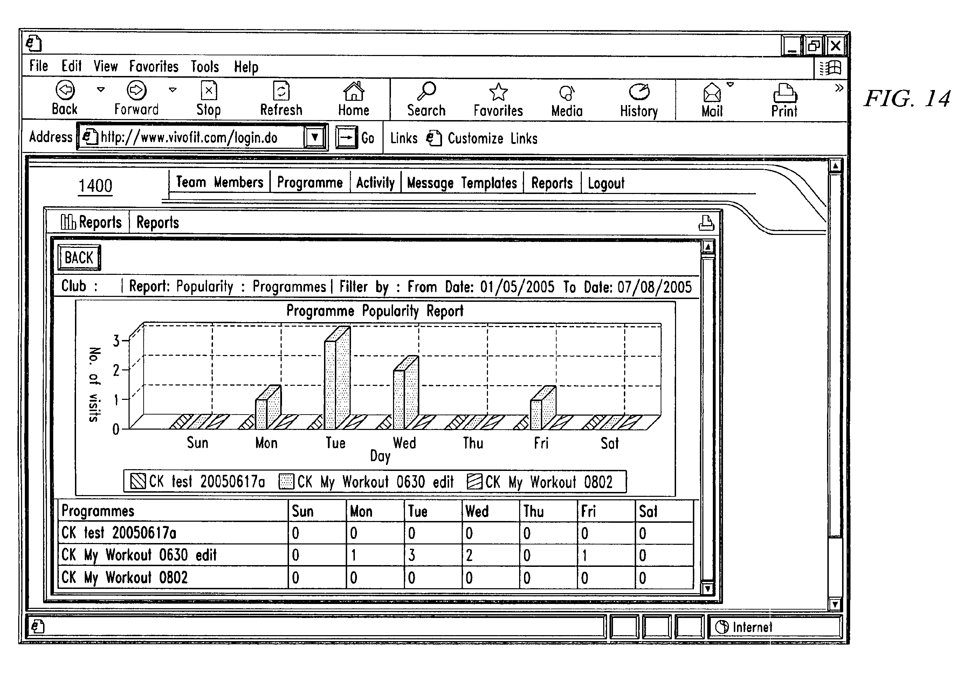

FIGS. 14 and 15 are example exercise machine monitoring user interfaces 1400 and 1500 associated with exercise machine monitoring processes. The example exercise machine monitoring user interface 1400 (i.e., the machine monitoring interface 1400) is a program popularity interface that indicates frequencies of use for exercise programs stored in the servers 112 and/or 130. The exercise programs may be, for example, exercise machine programs that are selected by fitness members via exercise machines (e.g., the exercise machines 120 and 122 of FIG. 1) or that fitness members incorporate into their workout schedules via, for example, the exercise template user interfaces 1000 and 1100. Although the machine monitoring interface 1400 is depicted in FIG. 14 as being associated with exercise machine programs, a program popularity interface that is substantially similar to the machine monitoring interface 1400 may be used to track or monitor fitness member participation in non-machine based exercises such as, for example, fitness classes, running track usage, pool usage, sauna usage, steam room usage, sports participation, racquetball court usage, etc. to determine the usage frequency of non-machine-based services, features, or programs.

The example exercise machine monitoring user interface 1500 (i.e., the machine monitoring interface 1500) depicts equipment popularity information indicating the usage frequency associated with exercise machines (e.g., the exercise machines 120 and 122) within a facility (e.g., the fitness facility 102, a rehabilitation center, a hospital, etc.) or a home. The information depicted in the machine monitoring interface 1500 may be generated based on usage information communicated to the service center 132 by the exercise machines 120 and 122 of FIG. 1, and 212 of FIG. 2. The information displayed via the machine monitoring interfaces 1400 and 1500 may be accessed by fitness facility personnel to develop and offer services and/or fitness facility features based on fitness member preferences.

Although certain methods, apparatus, and articles of manufacture have been described herein, the scope of coverage of this patent is not limited thereto. To the contrary, this patent covers all methods, apparatus, and articles of manufacture fairly falling within the scope of the appended claims either literally or under the doctrine of equivalents.

## Claims

1. A physical rehabilitation system comprising:
a first location comprising an exercise machine and a biometric sensor configured for use by a person; and
a second location comprising a first computing device communicatively coupled to the exercise machine and the biometric sensor, wherein the first computing device is configured to obtain information from the exercise machine and the biometric sensor, and to provide configuring data to the exercise machine, and
wherein the configuring data is determined by a medical professional in response to at least one of the obtained information, a medical history of the person, or a medical status of the person.

2. A system as defined in claim 1, wherein the medical professional is one of a physician, a family nurse practitioner, a nurse, a therapist, a physical therapist, a rehabilitation therapist, a doctor, a surgeon, a medical technician, or a medical equipment provider.

3. A system as defined in claim 1, wherein the first and second locations are geographically separated.

4. A system as defined in claim 1, wherein the configuring data defines an exercise routine for the exercise machine that is customized for the person.

5. A system as defined in claim 1, wherein the information provided by the biometric sensor includes physiological information associated with the person.

6. A system as defined in claim 1, wherein the information provided by the exercise machine includes usage information.

7. A system as defined in claim 1, wherein the first location further comprises a wireless network to communicatively couple the exercise machine and the biometric sensor to the second location via a wireless access point.

8. A system as defined in claim 1, wherein the first and second locations are communicatively coupled via an Internet Protocol based network.

9. A system as defined in claim 1, wherein the first location further comprises a second computing device communicatively coupled to at least one of the exercise machine or the biometric sensor.

10. A system as defined in claim 9, wherein the second computing device includes a touch screen to facilitate interaction with the second computing device.

11. A system as defined in claim 9, wherein the second computing device is configured so the person can at least one of view a prescribed workout plan, view a message from the medical professional, view a progress report, send a message to the medical professional, receive nutrition information, receive health information, receive a medical instruction, ask a question, request information, or receive an answer to a question.

12. A system as defined in claim 9, wherein the second computing device is configured to obtain the information from the exercise machine or the biometric sensor, and to provide the configuring data to the exercise machine.

13. A system as defined in claim 12, wherein the second computing device is configured to provide the obtained information to the first computing device and receive the configuring data from the first computing device.

14. A system as defined in claim 12, wherein the second computing device is configured to obtain the information and provide the configuring data when at least one of the exercise machine or the biometric sensor can not communicate with the first computing device.

15. A system as defined in claim 9, wherein the second computing device is configured to allow the medical professional or a different medical professional to at least one of determine the configuring data, review the obtained information, review the medical history, or review the medical status.

16. A system as defined in claim 9, wherein the second computing device comprises a wireless-enabled hand-held computing device.

17. A system as defined in claim 9, wherein the second computing device is configured to identify the person using at least one of a radio frequency identification device worn or carried by the person, a password entered by the person, an identification number entered by the person, a card reader device, or a finger print reader.

18. A system as defined in claim 1, further comprising
a third location communicatively coupled to the second location and comprising a second computing device configured to exchange with the first computing device at least one of the obtained information, the configuring data, the medical status of the person, the medical history of the person, person identification information or insurance information, wherein the second computing device is configured to be operated by at least one of a second medical professional, an employee of an insurance company, or a research scientist.

19. A system as defined in claim 1, wherein the exercise machine is configured to identify the person using at least one of a radio frequency identification device worn or carried by the person, a password entered by the person, an identification number entered by the person, a card reader device, or a finger print reader.

20. A physical rehabilitation system comprising:
an exercise machine at a first location configured for use by a person, to provide usage information to a first computing device not at the first location, and to respond to configuring data provided by the first computing device;
a biometric sensor at the first location configured for use by the person and to provide physiological data to the first computing device; and
a wireless network at the first location to communicatively couple the exercise machine and the biometric sensor to the first computing device, wherein the configuring data is determined by a medical professional based on at least one of the provided usage information, the provided physiological data, a medical history of the person, or a medical status of the person.

21. A system as defined in claim 20, wherein the physiological data includes at least one of heart rate information, heart rhythm information, breathing rate information, breathing rhythm information, blood oxygen level information, blood pressure information, body temperature information, or skin temperature information.

22. A system as defined in claim 20, wherein the biometric sensor is configured to be worn by, carried by, attached to, adhered to, or embedded within the person.

23. A system as defined in claim 20, further comprising a second computing device at the first location communicatively coupled via the wireless network to at least one of the exercise machine, the first computing device or the biometric sensor.

24. A system as defined in claim 23, wherein the second computing device is configured so the person can at least one of view a prescribed workout plan, view a message from the medical professional, view a progress report, send a message to the medical professional, or receive nutrition information, receive health information, receive a medical instruction, ask a question, request information, or receive an answer to a question.

25. A system as defined in claim 23, wherein the second computing device is configured to at least one of obtain the usage information from the exercise machine, obtain the physiological data from the biometric sensor, or provide the configuring data to the exercise machine.

26. A system as defined in claim 25, wherein the second computing device is configured to provide the configuring data when the exercise machine is not able to communicate with the first computing device.

27. A system as defined in claim 23, wherein the second computing device is configured to allow the medical professional or another medical professional to at least one of determine the configuring data for the exercise machine, review the usage information, review the physiological data, review the medical history, or review the medical status.

28. A system as defined in claim 20, wherein the exercise machine is configured to identify the person using at least one of a radio frequency identification device worn or carried by the person, a password entered by the person, an identification number entered by the person, a card reader device, or a finger print reader.

29. A physical rehabilitation system comprising
a computing device at a first location configured to:
receive biometric data associated with a person;
receive usage information of an exercise machine by the person; and
provide configuring data to the exercise machine, wherein the exercise machine and the person are not at the first location, and wherein the computing device is configured to enable another person to determine the configuring data in response to the received biometric data, the received usage data, a medical history for the person, or a medical status of the person.

30. A system as defined in claim 29, wherein
the computing device is configured to exchange at least one of the usage information, the biometric data, the configuring data, the medical status of the person, the medical history of the person, person identification information or insurance information with a second computing device not located at the first location; and
wherein the second computing device is configured to be operated by at least one of a medical professional, an employee of an insurance company, or a research scientist.

31. A method for providing a physical rehabilitation service comprising:
configuring an exercise machine and a physiological sensor for use by a person at a first location;
configuring a first computing device at a second location to receive information from the exercise machine and the physiological sensor and to provide configuring data to the exercise machine;
configuring a wireless network and wireless access point to couple the exercise machine and the physiological sensor to the first computing device; and
determining the configuring data responsive to a medical professional and at least one of the received information, a medical history of the person, or a medical status of the person.

32. A method as defined in claim 31, wherein the information provided by the physiological sensor is physiological information associated with the person and wherein the information provided by the exercise machine includes usage information.

33. A method as defined in claim 31, further comprising configuring a second computing device at the first location to be communicatively coupled to at least one of the exercise machine, the physiological sensor or the first computing device.

34. A method as defined in claim 33, further comprising configuring the second computing device so the person can at least one of view a prescribed workout plan, view a message from the medical professional, view a progress report, send a message to the medical professional, or receive nutrition information, receive health information, receive a medical instruction, ask a question, request information, or receive an answer to a question.

35. A method as defined in claim 34, further comprising configuring the second computing device to at least one of obtain the information from the exercise machine, to obtain the information from the physiological sensor, or to provide the configuring data to the exercise machine.

36. A method as defined in claim 35, further comprising:
configuring the second computing device to provide the configuring data to the exercise machine when the exercise machine is not communicatively coupled to the second location; and
configuring the exercise machine to respond to the configuring data from the first computing device or the second computing device.

37. A method as defined in claim 33, further comprising configuring the second computing device to allow the medical professional or a second medical professional to at least one of determine the configuring data for the exercise machine, review the obtained information, the medical history, or the medical status.

38. A method as defined in claim 31, further comprising
configuring a second computing device at a third location to exchange at least one of the obtained information, the configuring data, the medical status of the person, the medical history of the person, person identification information or insurance information with the first computing device; and
wherein the second computing device is configured to be operated by at least one of a second medical professional, an employee of an insurance company, or a research scientist.
